# EUROPEAN PATENT SPECIFICATION

(11) **EP 1 708 752 B1**
(45) Date of publication and mention of the grant of the patent: **22.02.2012**
(21) Application number: 05722768.8
(22) Date of filing: 27.01.2005
(51) Int. Cl.: A61K 39/395, A61K 47/48, A61P 35/02, C07K 16/28, C07K 16/30

(54) **POLYMER-BOUND ANTIBODY CANCER THERAPEUTIC AGENT**
THERAPEUTISCHES KREBSMITTEL MIT POLYMERGEBUNDENEM ANTIKÖRPER
AGENT THERAPEUTIQUE ANTICANCEREUX CONSTITUE D'ANTICORPS RELIE A UN POLYMERE

(30) Priority: 27.01.2004 US 539629 P
(43) Date of publication of application: 11.10.2006
(73) Proprietor: UNIVERSITY OF SOUTHERN CALIFORNIA, Los Angeles, CA 90007-4344 (US)
(72) Inventor: EPSTEIN, Alan, L., La Canada, CA 91011 (US); KHAWLI, Leslie, A., Arcadia, CA 91006 (US)
(74) Representative: Viering, Jentschura & Partner
(86) International application number: PCT/US2005/003676
(87) International publication number: WO 2005/072479

(56) References cited:
- WO-A-02/072011
- US-A- 4 256 724
- US-A- 4 434 150
- US-A- 5 510 121
- US-A- 6 113 906
- US-A1- 2002 004 587
- US-A1- 2002 006 404
- US-A1- 2005 181 985
- MATHAS S ET AL: "Anti-CD20- and B-cell receptor-mediated apoptosis: evidence for shared intracellular signaling pathways." CANCER RESEARCH 15 DEC 2000, vol. 60, no. 24, 15 December 2000 (2000-12-15), pages 7170-7176, XP002485215 ISSN: 0008-5472
- KIRPOTIN D ET AL: "STERICALLY STABILIZED ANTI-HER2 IMMUNOLIPOSOMES: DESIGN AND TARGETING TO HUMAN BREAST CANCER CELLS IN VITRO" BIOCHEMISTRY, AMERICAN CHEMICAL SOCIETY. EASTON, PA, US, vol. 36, no. 1, 1 January 1997 (1997-01-01), pages 66-75, XP002927736 ISSN: 0006-2960
- GHETIE M A ET AL: "Homodimers but not monomers of Rituxan (chimeric anti-CD20) induce apoptosis in human B-lymphoma cells and synergize with a chemotherapeutic agent and an immunotoxin." BLOOD 1 MAR 2001, vol. 97, no. 5, 1 March 2001 (2001-03-01), pages 1392-1398, XP002485216 ISSN: 0006-4971
- SHAN DAMING ET AL: "Apoptosis of malignant human B cells by ligation of CD20 with monoclonal antibodies" BLOOD, W.B.SAUNDERS COMPANY, ORLANDO, FL, vol. 91, no. 5, 1 March 1998 (1998-03-01), pages 1644-1652, XP002182861 ISSN: 0006-4971
- SHAN D ET AL: "SIGNALING EVENTS INVOLVED IN ANTI-CD20-INDUCED APOPTOSIS OF MALIGNANT HUMAN B CELLS" CANCER IMMUNOLOGY AND IMMUNOTHERAPY, BERLIN, DE, vol. 48, no. 12, 1 March 2000 (2000-03-01), pages 673-683, XP008036078 ISSN: 0340-7004
- GOECKERITZ B E ET AL: "MULTIVALENT CROSS-LINKING OF MEMBRANE IG SENSITIZES MURINE B CELLS TO A BROADER SPECTRUM OF CPG-CONTAINING OLIGODEOXYNUCLEOTIDE MOTIFSINCLUDING THEIR METHYLATED COUNTERPARTS, FOR STIMULATION OF PROLIFERATION AND IG SECRETION" INTERNATIONAL IMMUNOLOGY, OXFORD UNIVERSITY PRESS, GB, vol. 11, no. 10, 1 October 1999 (1999-10-01), pages 1693-1700, XP000938323 ISSN: 0953-8178
- GHETIE M-A ET AL: "HOMODIMERIZATION OF TUMOR-REACTIVE MONOCLONAL ANTIBODIES MARKEDLY INCREASES THEIR ABILITY TO INDUCE GROWTH ARREST OR APOPTOSIS OF TUMOR CELLS" PROCEEDINGS OF THE NATIONAL ACADEMY OF SCIENCES OF USA, NATIONAL ACADEMY OF SCIENCE, WASHINGTON, DC, vol. 94, 1 July 1997 (1997-07-01), pages 7509-7514, XP000941404 ISSN: 0027-8424
- ZHANG NAN ET AL: "Generation of rituximab polymer and evaluation of CD20 hyper-crosslinking-Induced apoptosis in non-Hodgkin's lymphomas" JOURNAL OF IMMUNOTHERAPY, vol. 27, no. 6, November 2004 (2004-11), page S12, XP008093425 & 19TH ANNUAL SCIENTIFIC MEETING OF THE INTERNATIONAL-SOCIETY-FOR-BIOLO GICAL-THERAPY-OF-CANCER; SAN FRANCISCO, CA, USA; NOVEMBER 04 -07, 2004 ISSN: 1524-9557
- HURWITZ E ET AL: "Inhibition of tumor growth by poly(ethylene glycol) derivatives of anti-ErbB2 antibodies" CANCER IMMUNOLOGY AND IMMUNOTHERAPY, BERLIN, DE, vol. 49, no. 4-5, 1 July 2000 (2000-07-01), pages 226-234, XP002254077 ISSN: 0340-7004
- LE GALL F ET AL: "Di-, tri- and tetrameric single chain Fv antibody fragments against human CD19: effect of valency on cell binding" FEBS LETTERS, ELSEVIER, AMSTERDAM, NL, vol. 453, no. 1-2, 18 June 1999 (1999-06-18), pages 164-168, XP004259822 ISSN: 0014-5793
- ZHANG NAN ET AL: "Generation of rituximab polymer may cause hyper-cross-linking-induced apoptosis in non-Hodgkin's lymphomas." CLINICAL CANCER RESEARCH : AN OFFICIAL JOURNAL OF THE AMERICAN ASSOCIATION FOR CANCER RESEARCH 15 AUG 2005, vol. 11, no. 16, 15 August 2005 (2005-08-15), pages 5971-5980, XP002485217 ISSN: 1078-0432
- TOBIN EVAN ET AL: "Combination immunotherapy with anti-CD20 and anti-HLA-DR monoclonal antibodies induces synergistic anti-lymphoma effects in human lymphoma cell lines." LEUKEMIA & LYMPHOMA MAY 2007, vol. 48, no. 5, May 2007 (2007-05), pages 944-956, XP008093367 ISSN: 1042-8194
- OMELYANENKO V ET AL: "HPMA copolymer-anticancer drug-OV-TL-TL16 antibody conjugates. 1. Influence of the method of synthesis on the biding affinity to OVCAR-3 ovarian carcinoma cells in vitro.", JOURNAL OF DRUG TARGETING, vol. 11, no. 5, June 2003 (2003-06), pages 295-309, ISSN: 1061-186X
- ULBRICH K ET AL: "Antibody-targeted polymer-doxorubicin conjugates with pH-controlled activation", JOURNAL OF DRUG TARGETING, HARWOOD ACADEMIC PUBLISHERS GMBH, DE, vol. 12, no. 8, 1 January 2004 (2004-01-01), pages 477-489, XP008087971, ISSN: 1061-186X
- D'SOUZA A J M ET AL: "Polyvinylpyrrolidone-drug conjugate: synthesis and release mechanism", JOURNAL OF CONTROLLED RELEASE, ELSEVIER, AMSTERDAM, NL LNKD- DOI:10.1016/J.JCONREL.2003.09.014, vol. 94, no. 1, 8 January 2004 (2004-01-08), pages 91-100, XP004480740, ISSN: 0168-3659
- D'SOUZA AJIT JOSEPH M ET AL: "Reaction of a peptide with polyvinylpyrrolidone in the solid state.", JOURNAL OF PHARMACEUTICAL SCIENCES MAR 2003 LNKD- PUBMED:12587120, vol. 92, no. 3, March 2003 (2003-03), pages 585-593, ISSN: 0022-3549
- NAGAPUDI K ET AL: "Amorphous active pharmaceutical ingredients in preclinical studies: Preparation, characterization, and formulation", CURRENT BIOACTIVE COMPOUNDS 2008 BENTHAM SCIENCE PUBLISHERS B.V. NLD LNKD- DOI:10.2174/157340708786847852, vol. 4, no. 4, 2008, pages 213-224, ISSN: 1573-4072
- LEUNER CHRISTIAN ET AL: "Improving drug solubility for oral delivery using solid dispersions", EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 1 July 2000 (2000-07-01), pages 47-60, XP002155099, ISSN: 0939-6411, DOI: DOI:10.1016/S0939-6411(00)00076-X
- GREENWALD R B: "PEG drugs: an overview", JOURNAL OF CONTROLLED RELEASE, ELSEVIER, AMSTERDAM, NL, vol. 74, no. 1-3, 6 July 2001 (2001-07-06) , pages 159-171, XP004297521, ISSN: 0168-3659, DOI: DOI:10.1016/S0168-3659(01)00331-5

## Description

### FIELD OF THE INVENTION

The present invention relates to a cancer therapeutic agent which comprises a polymer to which is bound multiple anti-tumor antibodies and uses of the agent for cancer therapy.

### BACKGROUND OF THE INVENTION

The following discussion of the background of the invention is merely provided to aid the reader in understanding the invention and is not admitted to describe or constitute prior art to the present invention.

Monoclonal antibodies (MAbs) have become an important treatment modality for human malignant lymphomas and other cancers. Three antibody products, RITUXAN, ZEVALIN, and BEXXAR, directed to the CD20 marker, have received FDA approval in the United States for lymphoma therapy. The use of these reagents is helping oncologists to design more effective treatment regimens.

CD20, which is a non-glycosylated 33-37 KD phosphoprotein expressed on >95% of normal and neoplastic B-cells (1). CD20 is expressed on the cell surface from the pre-B stage of development to terminal differentiation into plasma cells. Monocytes, resting and activated T-cells, null cells, and non-lymphoid cells are uniformly CD20 negative (2). The amino acid sequence of CD20 suggests that it is composed of four transmembrane-spanning regions with both the amino and carboxy terminus located on the cytoplasmic side of the plasma membrane (3). CD20 is currently believed to function as a calcium channel, to initiate intracellular signals, and to modulate cell growth and differentiation (4).

The expression of CD20 at high surface densities on malignant lymphoma cells has provided the rationale for the development of MAbs to this cell surface target. In addition, CD20 has not shown a tendency to internalize or modulate after antibody binding (5).

RITUXAN (generic name: Rituximab) is a chimeric monoclonal antibody directed against CD20. Clinically, administration of Rituximab induces objective therapeutic responses in 25-50% of patients (6). To understand the differential responses to Rituximab, efforts have been made to investigate the mechanisms underlying its anti-tumor activity and the relative contributions of each. Ligation of CD20 with anti-CD20 antibodies such as Rituximab has been shown to activate tyrosine and serine/threonine protein kinases, which are non-covalently associated with CD20, thereby inducing tyrosine phosphorylation of phospholipase C-gamma (PLCγ) (7,8). Moreover, cross-linking CD20 with an anti-CD20 secondary antibody (e.g. goat-anti-mouse antibody (GAM)) has been shown to mobilize calcium from intracellular stores (9). Interestingly, different anti-CD20 antibodies have varying effects on CD20 function. For instance, antibody IF5 stimulates B-cell cycle transition from G0 to G1 (10), whereas antibody B1 inhibits B-cell progression from the G1 phase of the cell cycle to the S/G2 + M stages following mitogen stimulation (4). These differences have now been shown to correlate with the ability of anti-CD20 antibodies to translocate the CD20 antigen into detergent-insoluble lipid microdomains, generally known as "rafts" which is required to trigger intracellular signals or activate lytic complement (11-14).

Harjunpaa et al. (15) and Manches et al. (16) have reported that direct complement killing is a fast and efficient effector mechanism of Rituximab administration, but recently, Weng and Levy (16) have provided differing results showing that the therapeutic activity of Rituximab does not correlate with the expression levels of the complement defense molecules CD55 or CD59. Because of these results, the role of CDC in Rituximab therapy has been questioned and the consumption of complement observed during Rituximab therapy may actually reflect toxicity rather than therapy.

The role of intracellular events that lead to apoptosis and cell death initiated by the cross-linking of CD20 either by Rituximab antibodies or FcR-bearing macrophages or other accessory cells that infiltrate tumors has been recently investigated (9,18,19). Clinical studies have shown that patients expressing the high-affinity variant of FcγRIIIa (158V allotype) respond better to Rituximab than those carrying the low-affinity 158F allotype (20). As a general mechanism, B-cell surface antigens and other antigens, including surface IgM (21,22), idiotype (23), CD19 (24), CD21 (28) CD22 (9) and HER-2 (28), and major histocompatibility complex (MHC) class II (25) have also been reported to initiate cell cycle arrest and/or apoptosis in mouse and human B-cells. Binding with anti-IgM or anti-MHC class II antibodies can directly induce apoptosis in B-lymphoma cells while anti-CD19, CD20, and CD22 antibodies induce apoptosis only if an additional step of cross-linking with a secondary antibody (e.g. goat anti-mouse antibody or GAM) is included (26). In CD20 expressing lymphoma cells, the induction of apoptosis by cross-linking with GAM antibodies was shown to be markedly reduced by Ca2+ chelation treatment with EGTA or Bapta AM, an intracellular calcium chelator (9).

### SUMMARY OF THE INVENTION

In one aspect, the present invention provides a cancer therapeutic agent according to claim 1.

As used herein, "tumor cell antigen" or "tumor antigen" refers to a protein which is present on tumor cells, and on normal cells during fetal life (onco-fetal antigens), after birth in selected organs, or on many normal cells, but at much lower concentration than on tumor cells. A tumor antigen is also referred to as a "tumor associated antigen" (TAA). A tumor antigen also includes what is referred to in the art as a tumor specific antigen (TSA) (aka. "tumor-specific transplantation antigen or TSTA). A TSA refers to a protein present on tumor cells but absent from non-tumor cells. TSA usually appear when an infecting virus has caused the cell to become immortal and to express virus antigens. TSAs not induced by viruses can be idiotypes of the immunoglobulin on B cell lymphomas or the T cell receptor (TCR) on T cell lymphomas. Tumor-associated antigens (TAA) are more common than TSA.

The antibodies selected for use in the therapeutic agent disclosed herein are characterized in their ability to induce apoptosis or cell death of tumor cells. In one embodiment, the antibodies are characterized in not substantially inducing apoptosis or cell death when directly applied to tumor cells, but inducing apoptosis or cell death when the tumor cell bound antibody is cross-linked by a secondary antibody (e.g. goat anti-mouse antibody or GAM; ref. 26) or by addition of FcR-bearing macrophages (the term "cross-linking" as used in the context of a secondary antibody, is also referred to in the art as "hyper-cross-linking"). Antibodies to CD19, CD20, CD21, CD22 and HER-2 are exemplary of this type of antibody. Such antibodies may be referred to as cross-link-dependent tumor cell apoptosis inducing antibodies. As described herein, a polymer to which such antibodies are linked exhibits enhanced ability to induce apoptosis or cell death as compared to the antibodies when not linked to the polymer.

Accordingly, disclosed herein is a method of converting an anti-tumor antibody that requires secondary cross-linking to induce substantial apoptosis or cell killing by the antibody into a cancer therapeutic agent that directly induces apoptosis and tumor cell killing. In accordance with this method, the antibody is conjugated to a polymer as disclosed herein.

In another embodiment, the antibodies which are linked to a polymer are characterized in inducing apoptosis or cell death following direct binding to tumor cells. Such antibodies include those that bind to surface IgM (21,22), idiotype (23), and major histocompatibility complex (MHC) class II antigens (25). MHC class II and idiotype may be considered a type of tumor associated antigen.

In one embodiment, the polymer comprises three or more such antibodies. In one embodiment, the polymer comprises at least 5, antibodies. In another embodiment, the polymer comprises at least 10 antibodies. In yet another embodiment, the polymer comprises at least 25 antibodies. In still another embodiment, the polymer comprises at least 50 antibodies. In still another embodiment, the polymer comprises at least 100 antibodies. In still yet another embodiment, the polymer comprise at least 300 antibodies, or comprises at least 1000 antibodies.

In one embodiment, the polymer comprises three or more antibody binding sites. In one embodiment, the polymer comprises at least 5 antibody binding sites. In another embodiment, the polymer comprises at least 10 antibody binding sites. In yet another embodiment, the polymer comprises at least 25 antibody binding sites. In still another embodiment, the polymer comprises at least 50 antibody binding sites. In still another embodiment, the polymer comprises at least 100 antibody binding sites. In still yet another embodiment, the polymer comprise at least 300 antibody binding sites, or comprises at least 1000 antibody binding sites.

As used herein, "polymer" means a naturally occurring or synthetic compound which is a large molecule made up of a linked series of repeated simple monomers. A polymer generally includes at least about 50 monomers. Natural polymers include protein, carbohydrate or nucleic acid, as well as non-natural water soluble biocompatible polymers. Non-natural polymers include water-soluble polymers such as polyethylene glycol, polypropylene glycol etc. Polymers may be substituted with functional groups such as hydroxy, amino, thiol, and carboxy.

In one embodiment, the polymer is at least 1,000 daltons in size. In another embodiment, the polymer is at least 2,000 daltons in size. In yet another embodiment, the polymer is at least 3,000 daltons in size. In still another embodiment, the polymer is at least 4,000 daltons in size. In still yet another embodiment, the polymer is at least 5,000 daltons in size. In a further embodiment, the polymer is at least 6,000 daltons in size. In yet another embodiment, the polymer is from 6,000 to 8,000 daltons in size. In a further embodiment, the polymer is between 1Kd to 30Kd.

In one embodiment, the polymer is a homopolymer. In another embodiment, the polymer is a heteropolymer. In yet another embodiment, the polymer is selected from the group consisting of dextran, block copolymers of poly(ethylene glycol)s (PEG), polyamidoamine dendrimers (PAPAM), synthetic copolymers of N-(2-hydroxypropyl)methacrylamide (HPMA), and nondegradable copolymers of N-(2-hydroxypropyl)methacrylamide (PHPMA).

In another embodiment, the polymer is a soluble polymer. In another embodiment, the polymer is a linear polymer. In yet another embodiment, the polymer is a branched polymer. In another embodiment, the polymer is insoluble.

In one embodiment, antibodies which bind to the same tumor antigen are conjugated to the polymer. If the antibodies are identical, the polymer is a homopolymer with respect to the antibody portion of the conjugate. If the antibodies are different, the polymer is a heteropolymer with respect to the antibody portion of the conjugate.

In another embodiment, antibodies which bind to different epitopes of the same tumor are conjugated to the polymer. In yet another embodiment, antibodies which bind to different tumor antigens are conjugated to the polymer. The latter two embodiments are heteropolymers with respect to the antibody portion of the conjugate. Thus, the polymer conjugate can comprise two or more different antibodies linked thereto. In any of these embodiments, the form of the antibody conjugated to the polymer may differ. Thus, the polymer may be conjugated with whole antibody and/or a fragment of an antibody. Also, the polymer may be conjugated with more than one type of antibody fragment.

In one embodiment, the anti-tumor antibody is selected from the group consisting of anti-CD19, anti-CD20, anti-CD21, anti-CD22. In one embodiment, the anti-CD20 antibody is Rituximab. As used herein, Rituximab is a chimeric anti-CD20 antibody derived from a transfectoma comprising anti-CD20 in TCAE 8 as deposited under ATCC No. 69119 (see U.S. patent no. 5,776,456). In another embodiment, the-tumor antibody is selected from the group consisting of an anti-lung, anti-breast, anti-colon, anti-ovarian, anti-prostate, and an anti-sarcoma.

In another aspect, the cancer therapeutic agents are used for cancer therapy. The cancer therapeutic agents can be used reduce the size or reduce the growth of a tumor in an individual comprising administering an effective amount of the agent wherein the agent targets to the tumor *in vivo*.

Also disclosed is a method of reducing or inhibiting the development of metastasis in an individual suffering from cancer, comprising administering an effective amount of a cancer therapeutic agent wherein the agent targets to metastatic cell *in vivo*.

In the above therapeutic methods, a particularly suitable cancer patient for treatment is one that exhibits a reduced level of antibody dependent cell mediated cytotoxicity as compared to that of a normal age-matched individual. The extent of ADCC can be measured as described (24,25).

In yet another aspect, disclosed is a method of identifying an antibody that exhibits an enhanced ability to induce tumor cell apoptosis when at least two such antibodies are linked to a polymer compared to the antibody not linked to a polymer. The method comprises linking a candidate antibody for which such testing is desired to insoluble particles and contacting the antibody linked particles to tumor cells which express an antigen to which the candidate antibody can bind. A suitable time later, the extent of apoptosis induced in the tumor cells is determined. This is compared to the extent of apoptosis induced in a separate batch of the same tumor cells which are contacted with the candidate antibody unlinked to any particles. In one embodiment, the enhanced ability to induce tumor cell apoptosis is determined as an increase of at least two fold, more preferably at least 3 fold using the antibody linked particles as compared to the antibody unlinked to any particles. In another embodiment, the particles are beads. In a further embodiment, the beads have a diameter of between about 2 to about 5 microns in length. More preferably, the diameter is about 3 microns in length.

As used herein, "about " means plus or minus 10%. As used herein, "substantially" means 10% or more, more preferably 20% or more, more preferably 30% or more, more preferably 40% or more, and more preferably 50% or more.

### BRIEF DESCRIPTION OF THE FIGURES

FIGs. 1A-1K show CD20 expression by FACS analysis among different established B-cell lymphoma and Hodgkin's Disease cell lines.

FIG. 2 shows Raji cell proliferation in suspension cultures in the presence of Lym-1 antibody and control IgG.

FIGs. 3A-3G show induction of apoptosis of Raji cells in suspension by Rituximab and Lym-1 antibody, with and without hypercross-linking with a secondary antibody (goat anti-human IgG GAH" for Ritiximab and goat anti mouse IgG "GAM" for LYM-1). Apoptosis was evaluated by Annexin V-FITC and propidium iodide (PI) counterstaining and FACS analysis. Staining of Raji cells with control IgG is shown for comparison.

FIGs. 4A-4C show DNA fragmentation in Raji cells in suspension induced by anti-lymphoma antibodies, LYM-1 and Rituximab, and control antibody (chTV-1). TUNEL assay with detection of BrdU generated DNA strand breaks stained with FITC labeled anti-BrdU antibody followed by FACS analysis.

FIGs. 5 A-5B show analysis of polymer antibodies by Coomassie blue-stained SDS-PAGE (non-reducing conditions). Lane MW (molecular weight standards 123Kd and 204Kd); Lane a: Rituximab; Lane b: Dextran-Rituximab; Lane c: Dextran-Lym-1; and Lane d; Lym-1.

FIG. 6 shows induction of apoptosis of Raji cell suspensions by control IgG, Rituximab, Dextran-Rituximab, LYM-1 and Dextran-LYM-1. Apoptosis demonstrated by Annexin V-FITC and propidium I staining and FACS analysis.

FIGs. 7A-7B show biodistribution of Rituximab and Dextran-Rituximab in Raji lymphoma-bearing nude mice three days following i.v. administration. Results are shown as percent injected dose/gram (FIG. 7A) and as tumor/normal organ ratios (FIG. 7B).

FIGs. 8A-8B show biodistribution of LYM-1 and Dextran-LYM-1 in Raji lymphoma-bearing nude mice three days following i.v. administration. Results are shown as percent injected dose/gram (FIG. 8A) and as tumor/normal organ ratios (FIG. 8B).

FIG. 9 shows Rituximab and Dextran-Rituximab immunotherapy in a Raji xenograft model. Nude mice with a 0.5 cm diameter subcutaneous tumor were treated 3x on alternate days (arrows, days 1, 3, and 5) by intravenous administration of control IgG, Rituximab or dextran-Rituximab conjugate. Tumor volume shown on the vertical axis and time on the horizontal axis. Day 1 indicates the time of initiation of treatment, which generally was 14 days post-tumor implantation.

FIG. 10 shows LYM-1 and Dextran-LYM-1 immunotherapy in Raji a xenograft model. Details are as described in FIG.9.

FIG. 11A shows apoptosis induced in Raji cells by treatment with control IgG, Rituximab, goat anti-human IgG ("GAH") and Rituximab + GAFF. Apoptosis determined by Annexin V-FITC and propidium iodide staining with FACS analysis.

FIGs. 11B1-B4 show apoptosis by the TUNEL assay for Raji cells treated the same as described in FIG. 11 A.

FIG. 12A shows apoptosis induced in Raji cells by treatment with Dynabeads, control IgG linked to Dynabeads, and Rituximab linked to Dynabeads. Apoptosis determined by Annexin V-FITC and propidium iodide staining with FACS analysis.

FIGs. 12B 1-B3 show apoptosis by the TUNEL assay for Raji cells treated the same as described in FIG. 12A.

FIGs. 13A-13C is show Caspase 3 activity in Raji cells at 5 hours (left hand graphs) and 24 hours (right hand graphs) following treatment with various antibodies. Cells were stained by fluorogenic protease substrate Phiphilux, (FL1-IT) and evaluated by FACS.

FIGs. 14A-14B show biodistribution of Rituximab, Rituximab dimer (dimer) and dextran-Rituximab polymer (polymer) in Raji lymphoma-bearing nude mice three days following i.v. administration. Results are shown as percent injected dose/gram (FIG. 14A) and as tumor/normal organ ratios (FIG. 14B).

FIG. 15 shows Rituximab, Rituximab dimer (dimer) and dextran-Rituximab polymer (polymer) and phosphate buffered saline (PBS) immunotherapy in a Raji xenograft model. Arrows indicate treatement by i.v. administration.

### DETAILED DESCRIPTION OF THE INVENTION .

In accordance with one aspect of the invention, cancer therapeutic agents according to claim 1 are provided.

As used herein "apoptosis" refers to physiologic or programmed cell death of cells characterized by specific morphological features, including cellular and nuclear pyknosis, cytoplasmic blebbing, and margination of condensed chromatin at the periphery of the nuclear envelop due to intranucleosomal DNA fragmentation and the production of DNA ladders which are visible by agar gel electrophoresis. See e.g. Ref. 27. Apoptotic cell killing occurs for a wide variety of cancers following treatment with particular anti-cancer agents. Apoptosis is an important regulatory mechanism for immune B-cells and is still fully functional after malignant transformation. Apoptosis can be measured as described herein and as reported previously (see e.g. U.S. Patent no. 6,368,596).

Binding of Rituximab antibody to CD20⁺ lymphoma cells fails to induce apoptosis to any significant extent (16). To enhance apoptosis, Rituximab can be hyper-crossed linked either by including a goat anti-human/mouse IgG, by addition of FcγR-bearing cells, or by immobilization of the antibody on plastic *in vitro* (16-19). Hyper-cross-linking of Rituximab redistributes CD20 into Triton X-insoluble cell membrane signaling-processing centers (20,21) followed by lipid raft clustering and transactivation of Src-family tyrosine kinases such as *Lyn, Fyn, and Lyc,* which further lead to apoptosis of the lymphoma cells (19,22-24).

Ghetie *et al.* (29) reported that tetravalent homodimers of Rituximab could induce higher apoptosis rates in human malignant lymphoma cell lines compared to monomer preparations obtained from the pharmacy. In these studies, apoptosis was observed at rates comparable to those obtained by goat anti-mouse hyper-cross-linking and, when tested in combination with chemotherapeutic agents such as Doxorubicin, homodimer preparations produced significantly better tumor cell killing.

Antibody-dependent cell-mediated cytotoxicity (ADCC), specifically Fc:FcR interaction, is considered the predominant effector mechanism of Rituximab therapy (25,26). Fcγ receptors on effector cells appear to be responsible for the *in vivo* activity of anti-CD20 antibodies in mice as evidenced by the use of knockout mouse models in which it has been shown that Fc:FcR interaction is required for the depletion of circulating and extravasated B lymphocytes (25). Current clinical studies are focusing on a dimorphism of the *FCGR3A* gene which encodes FcγRIIIa with either a phenylalanine (F) or a valine (V) at residue 158 which specifically reacts with the lower hinge region of IgG₁ with different affinities (V>F) (27-29). It is observed that homozygous FCGR3A-158V patients have a greater probability of experiencing clinical responses, supporting the critical function of the Fc:FcR interaction (27,29). For ADCC to be effective, it is assumed that Fc-receptor bearing cells must enter the tumor microenvironment to come in direct contact with its target. If this is the case, then Fc-receptor bearing cells may function both as mediators of ADCC and hyper-cross-linking induced apoptosis to destroy the tumor. Thus, a lack of tumor associated effector cells may explain Rituximab failure in particular patients. The use of antibody polymers of the invention are useful for therapeutic cancer intervention in individuals that have reduced ADCC capability.

The cancer therapeutic agents of the invention comprising antibodies such as Rituximab represent a high valency reagent which is a more potent inducer of apoptosis than the monomer or dimer preparations both by *in vitro* and *in vivo* tests. Because of its soluble characteristics, polymer-linked Rituximab is a clinically relevant reagent that has demonstrated good tumor targeting and therapeutic potential in CD20⁺ Raji xenografts. Soluble polymers containing multiple anti-tumor antibodies provide a significant improvement antibody based immunotherapy, especially in patients with low affinity FcγR allotypes.

Anti-tumor antibodies, characterized by failure to induce substantial apoptosis or cell death when not cross linked with a secondary antibody (or contacted with Fc receptor bearing macrophages) can be identified using a tritiated thymidine incorporation assay as described in U.S. Patent no. 6,368,596. Anti-tumor antibodies characterized by enhanced apoptosis or cell killing following secondary induced cross-linking include anti-CD19, anti-CD20, anti-CD21, anti-CD22 and HER-2. In one embodiment, the anti-CD20 antibody is Rituximab. Other antibodies include antibodies to any of various carcinomas including those of lung, breast, colon, ovarian, prostate, as well as sarcomas. Other such antibodies are described in U.S. Patent no. 6,368,596 or may be readily identified using the methods disclosed herein.

CD19 (aka. B4 antigen) is a 95 Kd B cell marker detected by anti-CD19 antibodies (e.g. antibody FMC63) which include chimeric forms of these antibodies. Zola et al. Immunol Cell Biol. 69 (Pt 6):411-22 (1991); Pieterxz et al. Cancer Immunol Immunother. 41(1):53-60 (1995). CD19 is B lineage-specific and is expressed on early B-cell precursors, pre-pre-B-cells, pre-B- cells, B-cells, intermediate B-cells, mature B-cells and some plasmacytoid cells. Plasma cells and myeloma cells are CD19 negative.

CD21 is a receptor (about 145 Kd) for complement fragments C3d, C3dg or ic3D and also EBV. CD21 is also a ligand for CD23 and is involved in IgE synthesis. Anti-CD21 antibodies have been described. Anti-CD21 antibody binding to B cells has been reported to protect the cells from undergoing apoptosis. Bonnefoy, et al. Eur. J. Immunol. 23, 969-972 (1993).

CD22 is a 130 Kd protein expressed on human B lymphocytes. Humanized antibodies (e.g. Epratuzumab, Amgen, CA) to CD22 have been described. Carnahan et al., Clin Cancer Res. 9(10 Pt 2):3982S-90S (2003). Normal T-cells, polymorphonuclear leukocytes, monocytes and platelets are negative for CD22. Cell lines SB, Raji and NALM-1 are positive as is CALLA-ALL. The expression pattern for CLL and NHL is variable from weak to negative. PLL is clearly positive and Hairy Cell Leukaemia is very strong positive.

The proto-oncogene Her-2/neu (C-erbB2) has been localized to chromosome 17q and encodes a transmembrane tyrosine kinase growth factor receptor. The protein product of the Her-2/neu gene is overexpressed in 25-30% of breast cancers, and in approximately 90-95% of these cases, upregulation is a direct result of gene amplification. Antibodies to HER2 can block the proliferation signaling pathways of tumor, resulting in inhibition of tumor growth and induction of tumor cell apoptosis. *See e.g.*, Clin. Cancer Res. 8:1720-30 (2002); Brodowicz et al. Br. J. Cancer 85:1764-70 (2001); Crombet-Ramos et al., Int. J. Cancer 101: 567-75 (2002); Herbst et al., Expert Opin. Biol. Ther. 1:719-32 (2001). HER-2 antibodies have been described previously (see U.S. Patent Nos. 6,054,561; 5, 169,774; 4,938,948; 4,753,894, 6,387,371; 6,399,063 6,627,196; 6,821,515; 6,719,971; and U.S. 6,800,738.

The term "protein" as used herein refers to one or more polypeptides that may be isolated from a natural source, or produced from an isolated cDNA using recombinant DNA technology. If more than one polypeptide are included in a protein, the polypeptides are held together as a single molecule (i.e. multimer) via covalent and/or non-covalent forces. An example of a protein made up of multiple polypeptides is an antibody which contains heavy and light chains. The term protein includes proteins which have other chemical entities such as glycoprotein, proteoglycan, lipoprotein and the like.

The term "nucleic acids" as used herein shall be generic to polydeoxyribonucleotides (containing 2'-deoxy-D-ribose or modified forms thereof), to polyribonucleotides (containing D-ribose or modified forms thereof), and to any other type of polynucleotide which is an N-glycoside of purine or pyrimidine bases, or modified purine or pyrimidine bases.

As used herein, the term "purified" in reference to polypeptides (or proteins) does not require absolute purity. Instead, it represents an indication that the polypeptide(s) of interest (e.g antibodies) is(are) in a discrete environment in which abundance (on a mass basis) relative to other proteins is greater than in a biological sample. By "discrete environment" is meant a single medium, such as a single solution, a single gel, a single precipitate, etc. Purified polypeptides may be obtained by a number of methods including, for example, laboratory synthesis, chromatography, preparative electrophoresis, centrifugation, precipitation, affinity purification, etc. One or more "purified" polypeptides of interest are preferably at least 10% of the protein content of the discrete environment. One or more "substantially purified" polypeptides are at least 50% of the protein content of the discrete environment, more preferably at least 75% of the protein content of the discrete environment, and most preferably at least 95% of the protein content of the discrete environment. Protein content may be determined using a modification of the method of Lowry et al., J. Biol. Chem. 193: 265, 1951, described by Hartree, Anal Biochem 48: 422-427 (1972), using bovine serum albumin as a protein standard.

Cancers treatable using the methods of the invention include carcinomas, sarcomas, and leukemias and lymphomas and other types of cancer. Carcinomas include those of lung, breast, colon, ovarian, prostate, and the like. A preferred cancer for treatment is a lymphoma.

The polymer to which antibody is conjugated is preferably a soluble polymer. It is preferably about 1,000 daltons or larger. The polymer can be a homopolymer or a heteropolymer. The polymer may be selected from dextran, block copolymers of poly(ethylene glycol)s (PEG), polyamidoamine dendrimers (PAPAM), synthetic copolymers of N-(2-hydroxypropyl)methacrylamide (HPMA), nondegradable copolymers of N-(2-hydroxypropyl)methacrylamide (PHPMA), and the like. See Ulbrich et al..(2002) Materials Structure 10 (1): 3. Water soluble non-antigenic polymers may be used as described in U.S. Patent no. 6,113,906. Water-soluble polymers carriers may be biodegradable. The Polymer may be a polypeptide.

Polymer may be from 1,000 up to 30,000 in molecular weight. Polymers preferably are about 6,000-8,000 daltons in size or less.

Antibody may be coupled to polymers using chemistries described herein and others well known in the art. Linking chemistries may replicate chemistry strategies for preparing hapten carrier conjugates or drug carrier conjugates where the antibody is analogous to the hapten or drug and the polymer analogous to the carrier.

Multiple peptide or protein molecules may be attached to a single modified polyethylene glycol (PEG) polymer as described in Jones et al., Bioconjugate Chem. (2003) 14, 1067-76. Specifically, one or both termini of a polyethylene glycol polymer are modified to include two, four or eight aminooxy groups each. Linking structures connecting four modified PEG polymer molecules to provide attachment for four peptide or protein molecules are also disclosed. The conjugates are prepared by forming oxime linkages between the aminooxy groups of the PEG and the N-tenminally glyoxylated domains of the polypeptides. Jones et al. described conjugates of B-cell epitopes of β2GPI for the purpose of inducing B-cell tolerance to β2GPI. Related U.S. Patent 6,458,953 describes similar compounds (with and without PEG) for use as multivalent platforms for attachment of biological molecules, including antibodies. The peptides or proteins may be attached to the platform via various linkages such as thioether linkages. However, the preparation of PEG polymer conjugated to anti-tumor cell antibodies which is useful for inducing apoptosis of tumor cells has not been described. PEG conjugation to antibodies also may be performed using approaches described in 5,670,132; 5,234,903 and 5,234,903.

Polymer conjugates of the present invention differ from those previously known in the nature of the antibodies used for conjugation and in the ratio of antibody to conjugate. In prior studies, polymer was conjugated to antibody but in ratios of more than one polymer per antibody. In contrast, polymer antibody conjugates of the present invention have more than one antibody per polymer. A single polymer comprises at least 5, 10,15, 20, 25, 30,35, 40, 45, 50, 60. 70, 80, 90, 100, 200, 300, 400, 500, 1000, or more antibodies. In another embodiment, a single polymer comprises at least 5,10, 15, 20, 25, 30, 35, 40, 45, 50, 60. 70, 80, 90, 100, 200, 300, 400, 500, 1000, or more antibody binding sites.

The term "antibody" as used herein includes immunoglobulins, which are the product of B cells and variants thereof as well as the T cell receptor (TcR), which is the product of T cells, and variants thereof. An immunoglobulin is a protein comprising one or more polypeptides substantially encoded by the immunoglobulin kappa and lambda, alpha, gamma, delta, epsilon and mu constant region genes, as well as myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Also subclasses of the heavy chain are known. For example, IgG heavy chains in humans can be any of IgG1, IgG2, IgG3 and IgG4 subclass.

A typical immunoglobulin structural unit is known to comprise a tetramer. Each tetramer is composed of two identical pairs of polypeptide chains, each pair having one "light" (about 25 kD) and one "heave" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively.

Antibodies exist as full length intact antibodies (bivalent) or as a number of well-characterized fragments produced by digestion with various peptidases or chemicals. Thus, for example, pepsin digests an antibody below the disulfide linkages in the hinge region to produce F(ab')2, a dimer of Fab which itself is a light chain joined to VH-CH1 by a disulfide bond. The F(ab')2 may be reduced under mild conditions to break the disulfide linkage in the hinge region thereby converting the F(ab')2 dimer into an Fab' monomer (monovalent). The Fab' monomer is essentially a Fab fragment with part of the hinge region (see, Fundamental Immunology, W. E. Paul, ed., Raven Press, N.Y. (1993), for a more detailed description of other antibody fragments). While various antibody fragments are defined in terms of the digestion of an intact antibody, one of skill will appreciate that any of a variety of antibody fragments may be synthesized de novo either chemically or by utilizing recombinant DNA methodology. Thus, the term antibody, as used herein also includes antibody fragments either produced by the modification of whole antibodies or synthesized de novo or antibodies and fragments obtained by using recombinant DNA methodologies.

Recombinant antibodies may be conventional full length antibodies, antibody fragments known from proteolytic digestion, unique antibody fragments such as Fv or single chain Fv (scFv), domain deleted antibodies, and the like. Fragments may include a domains or polypeptides with as little as one or a few amino acid deleted or mutated while more extensive deletion is possible such as deletion of one or more domains.

An Fv antibody is about 50 Kd in size and comprises the variable regions of the light and heavy chain. A single chain Fv ("scFv") polypeptide is a covalently linked VH::VL heterodimer which may be expressed from a nucleic acid including VH- and VL-encoding sequences either joined directly or joined by a peptide-encoding linker. See Huston, et al. (1988) Proc. Nat. Acad. Sci. USA, 85:5879-5883. A number of structures for converting the naturally aggregated, but chemically separated light and heavy polypeptide chains from an antibody V region into an scFv molecule which will fold into a three dimensional structure substantially similar to the structure of an antigen-binding site. See, e.g. U.S. Patent Nos. 5,091,513, 5,132,405 and 4,956,778.

An antibody may be a non-human antibody, a human antibody, a humanized antibody or a chimeric antibody, the latter comprising human and non-human antibody sequence. As is known in the art, chimeric antibody is prepared by exchanging a non-human constant region (heavy chain, light chain or both) with a human constant region antibody. See e.g. U.S. Patent No. 4,816, 567 to Cabilly et al. Methods of making humanized antibodies from non-human antibodies such as from murine antibodies are also well known (see, e.g., U.S. Patent No. 5,565,332 to Winter).

Antibodies may be linked to the polymer covalently or non-covalently. As used herein, "linked" means that under physiological conditions of pH; ionic strength and osmotic potential, the majority of the entities are associated with each other at equilibrium. Covalent linkage may be by any of a variety of chemicals cross-linking agents including, for example, homobifunctional or heterobifunctional cross-linking reagents, many of which are commercially available (see, e.g., Pierce Chemical Co. or Sigma Chemical Co.). Cross-linking can be achieved by any of a variety of chemistries well known in the art including, for example, activated polyethylene glycols, aldehydes, isocyanates, maleimides and the like. Further description of forming dimers and multimers from antibodies is provided in the Examples.

Antibodies may be prepared using recombinant expression methods such as in prokaryotic or eukaryotic cells as is well known in the art. (see e.g., U.S. Patent nos 5,116,943 and 6,331,415). In general, nucleic acid encoding the antibody can be cloned into an expression vector for high yield expression of the encoded product. The expression vector can be part of a plasmid, virus, or may be a nucleic acid fragment. The expression vector includes an expression cassette into which the nucleic acid encoding the antibody is cloned in operable association with a promoter and optionally an enhancer. The expression cassette may also include other features such as an origin of replication, and/or chromosome integration elements such as retroviral LTRs, or adeno associated viral (AAV) ITRs. If secretion of the antibody is desired, DNA encoding a signal sequence may be placed upstream of the nucleic acid encoding the mature amino acids of the antibody. DNA encoding a short protein sequence that could be used to facilitate later purification (e.g., a histidine tag) or assist in labeling the antibody may be included within or at the ends of the antibody encoding nucleic acid.

Cells suitable for replicating and for supporting recombinant expression of antibody are well known in the art. Such cells may be transfected or transduced as appropriate with the particular expression vector and large quantities of vector containing cells can be grown for seeding large scale fermenters to obtain sufficient quantities of the protein for clinical applications. Such cells may include prokaryotic microorganisms, such as *E. coli*, or various other eukaryotic cells, such as Chinese hamster ovary cells (CHO), insect cells, or the like. Standard technologies are known in the art to express foreign genes in these systems.

The cancer therapeutic agents can be used for treatment of cancer in an individual so afflicted. Accordingly, disclosed herein is a method of reducing the size or reducing the growth of a tumor in an individual comprising administering an effective amount of the invention composition wherein the cancer therapeutic agent localizes to cancer cells in the individual. Further disclosed is the a method of reducing or inhibiting the development of metastasis in an individual suffering from cancer, comprising administering an effective amount of the composition, wherein the cancer therapeutic agent localizes to metastatic cancer cells in the individual.

The effectiveness of treatment using the cancer therapeutic agents may be increased by depleting or inactivating immunoregulatory T cells in the individual. The term "immunoregulatory T cells" as used herein refers to a population of T cells that function, directly or indirectly, to suppress the immune response of a host to a tumor. Immunoregulatory T cells may be CD4⁺, CD25⁺ or positive for both markers.

The terms "depleting or inactivating" as used herein refer to methods that are effective in reducing suppression of the immune system mediated by immunoregulatory T cells. Such methods may remove cells from the individual (deplete) or may inactivate the cell function that results in suppression of the anti-tumor immune response. Methods of depleting or inactivating immunoregulatory T cells need not be limited solely to such cells but may act on other type of immune cells.

Immunoregulatory T cells are depleted or inactivated by any of a variety of methods well known in the art. For example, such cells may be removed from the circulation of an individual by flow cytometry or by immunoadsorbant cell removal using, for example, antibody loaded columns or filters. Such columns or filters contain antibody specific for the CD4 antigen and/or antibody specific for the IL-2 receptor (e.g. CD25). Depleting or inactivating immunoregulatory T cells also may be performed by administering anti-CD4 and/or anti-CD25 specific antibodies to the individual. One or more antibodies may be used and preferably are human antibodies or humanized antibodies. For example, Daclizumab, a humanized monoclonal antibody, that binds to the alpha subunit of the IL-2 receptor (CD25) or basiliximab (chimeric antibody) may be used (both from Novartis Pharma AG). HuMax-CD4, a fully human antibody, is prepared by GenMab. Anti-CD40 ligand also may be used to deplete or inactivate immunoregulatory T cells.

A treatment to deplete or inactivate immunoregulatory T cells may be repeated. Also, different methods may be used together (immunoadsorbant cell removal and *in vivo* antibody administration). The amount of anti-T cell antibody administered for depletion or inactivation may be similar to the amount used in the transplantation field. See, e.g., Meiser et al., Transplantation. (1994) 27; 58(4): 419-23.

Immunoregulatory T cells may be depleted or inactivated before, during and/or after administration of the invention cancer therapeutic agents. Immunoregulatory T cells are preferably depleted before administering the invention cancer therapeutic agents.

In a further embodiment, the disclosed methods for cancer therapy may include adoptive transfer of immune cells to enhance anti-tumor immunity. These are preferably T cells, which may be activated *ex vivo* to enhance their ability to function in supporting an anti-tumor immune response. Adoptively transferred immune cells may be activated by *ex vivo* any of a variety of well known agents including, for example, exposure to IL-2 and/or to anti-CD3 antibodies. *Ex vivo* activation also may include exposure to a cancer cell vaccine. Such cancer cell vaccine may constitute live (but non-replicating), or killed cancer cells from the individual to be treated or from another cancer entirely. The vaccine also may be a cancer cell extract or purified vaccine preparation derived from cancer cells. Cancer cell vaccines are well known in the art and may be prepared in accordance with well known methods.

Adoptively transferred T cells may be administered before, during and/or after administration of the cancer targeting agent invention composition. Adoptively transferred T cells are preferably given after administration of the cancer targeting agent invention composition. In this form of therapy, patients receive multiple infusions of T-cells after *ex vivo* stimulation with IL-2 (62) or other agents such as anti-CD3⁺ and anti-CD28⁺ antibodies (63).

In some embodiments, the effectiveness of the therapy using the cancer therapeutic agents can be increased by combining the approach of depleting or inactivating immunoregulatory T cells with adoptive transfer of immune cells. In such embodiments it may be advantageous to adoptively transfer after the step of depleting or inactivating immunoregulatory T cells.

The cancer therapeutic agents can be administered as a pharmaceutical or medicament formulated with a pharmaceutically acceptable carrier. Accordingly, the compounds may be used in the manufacture of a medicament or pharmaceutical composition. Pharmaceutical compositions of the invention may be formulated as solutions or lyophilized powders for parenteral administration. Powders may be reconstituted by addition of a suitable diluent or other pharmaceutically acceptable carrier prior to use. Liquid formulations may be buffered, isotonic, aqueous solutions. Powders also may be sprayed in dry form. Examples of suitable diluents are normal isotonic saline solution, standard 5% dextrose in water, or buffered sodium or ammonium acetate solution. Such formulations are especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride, sodium citrate, and the like.

Alternately, compounds may be encapsulated, tableted or prepared in an emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier varies but, preferably, will be between about 20 mg to about 1 g per dosage unit. The pharmaceutical preparations are made following the conventional techniques of pharmacy involving milling, mixing, granulating, and compressing, when necessary, for tablet forms; or milling, mixing and filling for hard gelatin capsule forms. When a liquid carrier is used, the preparation may be in the form of a syrup, elixir, emulsion, or an aqueous or non-aqueous suspension. For rectal administration, the invention compounds may be combined with excipients such as cocoa butter, glycerin, gelatin or polyethylene glycols and molded into a suppository.

Compounds disclosed herein may be formulated to include other medically useful drugs or biological agents. The compounds also may be administered in conjunction with the administration of other drugs or biological agents useful for the disease or condition to that the compounds are directed. For example, the cancer therapeutic agents may be combined with another apoptotic inducing agent such as radiation or a chemotherapeutic agent (e.g. Doxorubicin; see, e.g. U.S. Patent no. 6,090,365).

As employed herein, the phrase "an effective amount," refers to a dose sufficient to provide concentrations high enough to impart a beneficial effect on the recipient thereof. The specific therapeutically effective dose level for any particular subject will depend upon a variety of factors including the disorder being treated, the severity of the disorder, the activity of the specific compound, the route of administration, the rate of clearance of the compound, the duration of treatment, the drugs used in combination or coincident with the compound, the age, body weight, sex, diet, and general health of the subject, and like factors well known in the medical arts and sciences. Various general considerations taken into account in determining the "therapeutically effective amount" are known to those of skill in the art and are described, e.g., in Gilman et al., eds., Goodman And Gilman's: The Pharmacological Bases of Therapeutics, 8th ed., Pergamon Press, 1990; and Remington's Pharmaceutical Sciences, 17the ed., Mack Publishing Co., Easton, Pa., 1990. Dosage levels typically fall in the range of about 0.001 up to 100 mg/kg/day; with levels in the range of about 0.05 up to 10 mg/kg/day are generally applicable. A compound can be administered parenterally, such as intravascularly, intravenously, intraarterially, intramuscularly, subcutaneously, or the like. Administration can also be orally, nasally, rectally, transdermally or inhalationally via an aerosol. The composition may be administered as a bolus, or slowly infused.

A therapeutically effective dose can be estimated initially from cell culture assays by determining an IC50. A dose can then be formulated in animal models to achieve a circulating plasma concentration range that includes the IC50 as determined in cell culture. Such information can be used to more accurately determine useful doses in humans. Levels in plasma may be measured, for example, by HPLC. The exact formulation, route of administration and dosage can be chosen by the individual physician in view of the patient's condition.

The following examples serve to illustrate the present invention. These examples are in no way intended to limit the scope of the invention.

### EXAMPLES

*Example 1: Materials and methods*

*1. Reagents.*

Molecular biology reagents were purchased from New England BioLabs (Beverly, MA) or Boehringer Mannheim (Indianapolis, IN). Immunological and crosslinker reagents were purchased from Sigma-Aldrich Chemical Co. (St. Louis, MO) or Pierce Biotechnology (Rockford, IL). Characterized and diaylyzed fetal calf sera were purchased from Hyclone Laboratories (Logan, Utah). Iodine-125 and Iodine-131 were obtained from DuPont/New England Nuclear (North Billerica, MA). BALB/c and athymic nude mice were purchased from Harlan Sprague Dawley (San Diego, CA).

*2. Antibodies and Cell Lines.*

The chimeric anti-HLA-Dr MAb (chLym-1) was produced as described above according to published results (41). The chimeric anti-CD20 MAb (Rituximab) developed by IDEC Pharmaceuticals (San Diego, CA) was obtained from the pharmacy. Goat anti-human IgG (Fc specific) for the hyper-cross-linking experiments was purchased from Caltag Laboratories (Burlingame, CA); Fluorescein isothiocyanate (FITC)-labeled goat anti-mouse F(ab')₂ used in the immunofluorescence microscopy studies was purchased from ICN-Cappel (Aurora, OH), and Phycoerythrin (PE) labeled anti-CD20 antibody used to determine CD20 expression was purchased from BD Pharmingen (San Diego, CA).

The Raji cell line (42) and other North American and African Burkitt's lymphoma cell lines (B35M, DG-75, RAMOS, Chevallier) were obtained from the American Type Culture Collection (Rockville, MD). The Farage, Granda 519, and L540 lymphoma and Hodgkin's Disease cell lines were obtained from DSMZ German Collection (Braunschweig, Germany). The SU-DHL-4, SU-DHL-6, SU-DHL-10, SU-DHL-16 and NU-DHL-1 human B-lymphoma cell lines were developed as previously described (43,44). All cell lines were grown in RPMI 1640 (Life Technologies, San Diego, CA) supplemented with 10% characterized fetal calf serum (FCS) (Hyclone, Logan, Utah), 1% Glutamine, and Penicillin (100 units/mL) and Streptomycin (100µg/mL) (Gemini Bio-Products, Woodland, CA, USA) antibiotics, and cultured in a humidified 5% CO₂ incubator at 37°C as stationary suspension cultures.

*3. Antibody Hyper-cross-linking.*

Raji cells (2.5x 10⁴) were incubated in a V-bottom 96-well plate with serial dilutions of Lym-1, chLym-1, Rituximab or other antibody at 37°C in a 5% CO₂ incubator for 1 hr. The cells were then washed with RPMI 1640 twice and divided into experimental and control groups. The cells in the experimental group were then incubated with 25ug/ml goat-anti-human (GAH) IgG (for chimeric Lye-1 and Rituximab MAbs) or goat-anti-mouse (GAM) IgG (for murine Lym-1) in a volume of 100µl/well. Eighteen hrs later, phosphotidylserine (PS) exposure on apoptotic cells was measured as described below using Annexin V-FITC/PI staining.

*4. Cross-linking Analysis by Immunofluorescence Microscopy*

The degree and pattern of antigen cross-linking on the surfaces of cells treated with different antibody preparations were observed by immunofluorescence microscopy. For these studies, 5 × 10⁵ Raji cells were treated with 20µg of each antibody preparation at 37°C, fixed with 2% paraformaldehyde (Polysciencs, Inc. Warrington, PA; Cat. No. 4018) diluted in PBS at room temperature followed by 2 washes with PBS (1% BSA). Fixed cells were then incubated with 25µg of GAM F(ab')₂ (ICN-Cappel) at 4°C for 30 min and subsequently washed twice with PBS (1% BSA). For the hyper-cross-linking studies by a secondary goat-anti-mouse IgG F(ab')_{2,} 5 x 10⁵ Raji cells were treated with 20µg of Rituximab for 1h at 37°C. The cells were washed with PBS twice and incubated with 25µg of GAM F(ab')₂ for 30 min at 4°C. Cells were then washed with PBS (1 % BSA) twice, followed by fixation using 2% paraformaldehyde at room temperature. Subsequently, the cells were washed twice with PBS. Finally, cytospin preparations were made at 1,250 x g for 10 min, and slides were mounted in Vectorshield with 4',6'-diamino-2-phenylindol (DAPI, Vector Laboratories, Burlingame, CA) for observation under a Phase Contrast Fluorescence Microscope (Leitz Orthoplan, Wetzlar, Germany) using a 50x water immersion lens.

*5. Caspase 3 Activity Assay*

Two µg ofRituximab monomer, dimer, and polymer, and Rituximab and isotype control antibody conjugated Dynabeads were incubated with 5 x 10⁴. Raji cells. Five and 24h later, the cells were washed with PBS, resuspended in 40µl of the fluorogenic protease substrate Phiphilux (OncoImmunin Inc., MD), and incubated in a 30°C waterbath for 40 min. Lastly, the cells were resuspended in media provided by the manufacturer and immediately analyzed by flow cytometry.

*6. Preparation of Multimers by Using Multifunctional Cross-linkers.*

Multi-functional cross-linkers are water-soluble reagents capable of multimeric aggregate formation (Table 1). Cross-linkers such as glutaraldehyde (45,46), dextran (47-49) and/or polyamidoamine (PAPAM) dendrimers (50-54) can be used to produce polymers of MAbs.

*A. Glutaraldehyde* is a five-carbon dialdehyde useful in a variety of protein applications as a cross-linker. Nucleophilic groups such as sulfhydryl and amino groups of the antibody are covalently added to the aldehyde groups forming a Schiff base in a one-step reaction. Briefly, the antibody (Rituximab, 1ml) is dialysed in the dark at a concentration of 5mg/ml against 200ml 8% glutaraldehyde in PBS (0.01M phosphate buffer saline, pH 7.2) for 16h at 4°C. The activated antibody is then dialysed against PBS (twice in 1L for several hours) to remove excess glutaraldehyde. The remaining active glutaraldehyde groups are blocked by 0.2M lysine-HCl (0.1ml) during a 2h incubation. The excess lysine is removed by dialysis against PBS. The cross-linked conjugate is then sterile filtered and further analyzed by SDS-PAGE.

*B. Dextran* is a polymer of glucose with an average molecular weight of 1 to 500k. The vicinal hydroxyls of dextran can be oxidized to aldehyde groups which are then reacted with the amino groups forming a Schiff base.

*Method 1: Polyaldehyde-Activated Dextran* (1: Rituximab: oxidized dextran). The binding of dextran to the antibody is performed by the use of the polyaldehyde-activated dextran as previously described (47). This can successfully act as a polyfunctional cross-linking agent and react with the primary amines of MAbs forming a Schiffbase that can be further stabilized by reduction with sodium borohydride. Briefly, dextran (100mg, average MW 6,000, Fluka) and sodium periodate (120mg, Sigma-Aldrich) are dissolved in 5ml of a 0.9% NaCl solution. After overnight incubation in the dark, the mixture is passed through a pre-equilibrated PD-10 column (Pharmacia) and eluted with 0.9% NaCl. The purified oxidized dextran is stored at 4°C, and the mixture is stable for 2 weeks at 4°C in the dark. Meanwhile, 1ml of purified Rituximab (10mg/ml) is passed through a pre-equilibrated PD-10 column and eluted with 0.1M Sodium Bicarbonate Buffer (pH 8.1). The eluted Rituximab is diluted to 4.2mg/ml with 0.1M Sodium Bicarbonate Buffer (pH 8.1), and then mixed with oxidized dextran at a 1:1 molar ratio. After continuous shaking 4h in the dark, the reaction is reduced with 5mg sodium borohydride (Sigma) for 1 h. Finally, 1ml of the Dextran-Rituximab conjugation is passed through a pre-equilibrated PD-10 column. The OD value of this conjugation was determined by a UV-spectrophotometer according to the antibody's concentration. The cross-linking efficiency is then determined by a non-reducing SDS PAGE.

*Method 2: Polyaldehyde-Activated Antibody.* A different method of covalent binding takes advantage of the sugar moiety of the antibody. This is performed in a two-step reaction by the coupling of dextran-hydrazine complex to polyaldehyde-activated MAbs. Briefly, the purified polyaldehyde-activated dextran (as above) is mixed with 0.09g hydrazine monohydrochloride and incubated for 30min at RT in the dark. The dextran-hydrazine derivative is mixed with trimethylamine borane complex (TMAB, 5mg) and incubated for an additional 2h at RT. Finally, the dextran-hydrazine derivative is passed through a pre-equilibrated PD-10 equilibrated with 0.1M NaAc pH 5.5. The polyaldehyde-activated Rituximab is generated by mixing 300µl of purified Rituximab (10mg/ml) with sodium periodate (126mg) in 1ml PBS pH 7.5. After continuous shaking for 90min at RT, the oxidized RITUXAMAB is purified on a PD-10 column (7.14mg/ml in PBS) and mixed with the dextran-hydrazine complex (82µl) for 18h at RT in the dark. The remaining active groups are blocked with 0.1M glycine pH7.5 (300µl) during a 2h incubation. The excess glycine is removed by dialysis against PBS and the cross-linked conjugate is sterile filtered and further analyzed by SDS-PAGE.

*C. PAPAM* is a new class of highly branched spherical polymers that are highly water-soluble and have large number of surface primary amino groups (50-54). The conjugation of the PAPAM dendrimers to the antibody is performed by the use of the different generation of dendrimers which can be successfully prepared in a two-step reaction procedure. Briefly, various generation dendrimers (G1 to G4) that possess 8 to 64 reactive amino groups on its surface (average MW 1.7 to 14.5k) are dissolved in PBS and mixed with a heterobifunctional cross-linking reagent. The resulting polymeric conjugates is dialyzed overnight, purified by chromatography on a Sephadex G-25, analyzed by SDS/PAGE and HPLC, and further characterized to determine the number of antibody molecules per dendrimer molecule.

**Table 1. Selected Multifunctional Cross-linking Reagents**

| **Reagent** | **Chemical Name** |
|---|---|
| Glutaraldehyde | Glutaric dialdehyde |
| Dextran | Dextran (average MW 1.7 to 14.5k) |
| PAPAM | Polyamidoamine dendrimers (average MW 1.7 to 14.5k) |

*7. In.Vitro Assessment of Monomer and Cross-linked Preparations*

A. Apoptosis assays.

i. Annexin V, PI Staining. The percentage of apoptosis induced by the antibody conjugations were determined by the Annexin V, PI staining assay which allows the direct detection of phosphatidylserine (PS) exposure on the apoptotic cells and loss of membrane integrity. Serial dilutions (about 2 µg) of Lym-1, chLym-1, and Rituximab or Rituximab coated Dynabeads (discussed *infra*) were added to 2.5 x 10⁴ Raji cells followed by an 18-hour incubation at 37°C. For hyper-cross-linking, 5 × 10⁴ Raji cells in 200µL media were incubated with 2µg of Rituximab for 1h at 37°C. The cells were then washed twice with PBS (1% BSA) and then incubated with 5µg of GAH (Caltag) for 18h at 37°C in a 5% CO₂ incubator. Annexin V-FITC and PI staining were performed using the RAPID protocol provided by the manufacturer (Oncogene Research Product, Boston, MA, USA). Briefly, Media Binding Reagent was added to the cells followed by a 15-minute incubation with FITC-labeled Annexin V. The cells were then resuspended in 1x Binding Buffer and propidium iodide (PI) was added just prior to analysis of the samples by flow cytometry (FACS-diva, Becton Dickinson, San Jose, CA, USA) to distinguish early (Annexin V+/PI-) and late (Annexin V+/PI+) apoptotic cells.

ii. TUNEL Assay. This assay was performed to measure apoptotic DNA strand breaks induced by the different antibody preparations using the APO-DIRECT Kit (Phoenix Flow Systems, San Diego, CA, USA). Briefly, 5 × 10⁵ to 1x 10⁶ Raji cells were incubated with 667ng/ml to 66.7ug/ml of each of MAb for 18h at 37°C in a 5% CO₂ incubator. For the hyper-cross-linking studies, 5 × 10⁵ Raji cells were incubated with 20µg of Rituximab in 1.5mL for 1h at 37°C. Cells were then washed twice with PBS and further incubated with 50µg of GAH (Caltag) in 1.5mL for 18h at 37°C in a 5% CO₂ incubator. Cells were washed in PBS, fixed in 1% paraformaldehyde, and permeabilized in 70% ethanol. The cells were stored in 70% ethanol overnight at -20°C. Bromodeoxyuridine triphosphate (BrdU) was coupled to the DNA strand breaks using terminal deoxynucleotidyl transferase (TdT) and fluorescein-labeled anti-BrdU antibody was then used to stain the apoptotic cells for FACS analysis. Total DNA in the cells was determined by counterstaining with PI/RNase. For each sample, 10,000 cells were analyzed by flow cytometry.

*8. Evaluation of CD20 and HLA-Dr Antigen Expression.*

Several different human malignant lymphoma (Raji, RAMOS, DG-75, Farage, Granda 519, Chevallier, SU-DHL-4, SU-DHL-10, SU-DHL-16) and a Hodgkin's Disease cells line (L540) positive for CD20 and HLA-DR10 antigens were assessed by FACs analysis to demonstrate the expression levels of these antigens on the surface of the lymphoma cells using standard methods.

*A. Proliferation Assays.* All antibody preparations were evaluated by a cell proliferation assay. For this assay, Raji cells (7.5x10^{4/}/well) suspended in RPMI-1640 medium (Life Technologies, Inc., San Diego, CA) containing 10% characterized fetal calf serum (Hyclone Laboratories, Logan, UT) were cultured with 67µg/ml of antibody. Cell proliferation was assessed by trypan blue dye exclusion at various time points using a hemocytometer. Four different fields of the hemocytometer were counted and averaged to obtain the data points.

*B. Determination of Avidity Constants.* Purified MAb preparations were radiolabeled with ¹²⁵I using a modified chloramine-T method as described previously (56). The *in vitro* immunoreactivities of radiolabeled proteins were evaluated by a conventional fixed Raji cell radioimmunoassay (55). Briefly, Raji lymphoma cell suspensions containing 10⁶ cells/mL were incubated with 10 to 110ng of ¹²⁵I-labeled antibody in 200µl PBS for 1h at room temperature with constant mixing. The cells were then washed 3x with PBS containing 1% bovine serum albumin to remove unbound antibody and counted in a gamma counter. The amount of antibody bound was then determined by the remaining cell-bound radioactivity (cpm) in each tube and the specific activity (cpm/ng) of the radiolabeled antibody. Scatchard plot analysis was used to obtain the slope. The equilibrium or avidity constant Kₐ was calculated by the equation K=-(slope/*n*), where *n* is the valence of the antibody (*n*=2 for the monomer; *n=4* for the dimer; n=*10* for the polymer).

*C*. *In Vitro Stability. In vitro* serum stability was also evaluated as described previously (56). Briefly, radioiodinated preparations are incubated for 48h in mouse and/or human serum at 37°C. After trichloroacetic acid precipitation and centrifugation, protein-bound radioactivity is measured in a gamma counter in order to calculate the percentage of intact fusion protein. In addition, the *in vitro* immunoreactivities of radiolabeled MAbs before and after incubation in serum are determined as described above.

9. *In Vivo* Assessment of Cross-Linked Preparations.

The biologic activity of the those preparations able to induce effective apoptosis *in vitro* were studied *in vivo* to determine their pharmacokinetic clearance and biodistribution. In addition, the anti-tumor activity of each reagent was studied in Raji human lymphoma-bearing nude mice by assessing their effects on tumor growth (tumor size, survival times) and morphology.

*A. Pharmacokinetic Studies.* ^{125/131}I-labeled proteins were prepared using a modified chloramine-T method as described previously (41,56). Six-week-old BALB/c mice were used to determine the whole-body pharmacokinetic clearance of all preparations. Groups of mice (n=5) previously fed potassium iodine in the drinking water for 1 wk to block thyroid uptake of radioiodine were administered i.v. injections of ¹²⁵I-labeled proteins (30-40µCi/mouse). The whole-body activity immediately post-injection and at selected times thereafter were measured with a CRC-7 microdosimeter (Capintec, Inc., Pittsburgh, PA). The data were analyzed and half-life values determined as described previously (41,57,58).

*B. Biodistribution Studies.* Tissue biodistribution studies were performed in Raji tumor-bearing nude mice to examine the targeting ability of each selected preparation. For these studies, six-week-old female athymic nude mice were irradiated with 350 rads from a cesium source and three days later were injected subcutaneously with a 0.2ml inoculum containing 1x10⁷ Raji lymphoma cells and 1x10⁶ human fetal fibroblast feeder cells in the left flank. Prior radiation was necessary to reduce natural killers cells present in the nude mice. The human fetal fibroblast feeder cells provide VEGF and other growth factors that promote a high tumor take rate (90%). The tumors were grown for 10-15 days until they reached 0.5 cm in diameter. Within each group (*n*=5), individual mice were injected i.v. with a 0.1 ml inoculum containing 30-40µCi of ¹²⁵I-labeled proteins. Animals were sacrificed by sodium pentobarbital overdose at 3 different time points post-injection (24, 48 and 72h), and tissues were removed, weighed, and measured in a gamma counter. For each mouse, data were expressed as percentage injected dose/gram (% ID/g) and as tumor/organ ratio (cpm per gram tumor/cpm per gram organ). Significance levels were determined using the Wilcoxon's rank-sum test.

*C. Therapy Studies:* For these studies, the anti-tumor activity of selected preparations were compared with that of unconjugated Lym-1 and RITUXAN to help evaluate which of the conjugated preparations were most effective in the Raji lymphoma/nude mouse model. Two doses (25 and 100µg) in a 0.1 ml inoculum for each preparation were administered to groups of mice (n=5) every other day X3 and tumor volumes were monitored 3x/week by caliper measurement performed in three dimensions. Regression or inhibition of tumor growth relative to untreated controls was determined to indicate the efficacy of therapy. All doses were administered by the same technician to maintain consistency. Animal survival times were recorded and those mice with tumors larger than 2 cm in diameter were sacrificed according to established University Vivarium protocols. Statistical analyses were performed using the Wilcoxin's rank sum test to obtain P values.

*Example 2*: *Results*

1. Expression levels of CD20 on human malignant lymphoma cell lines by FACs.

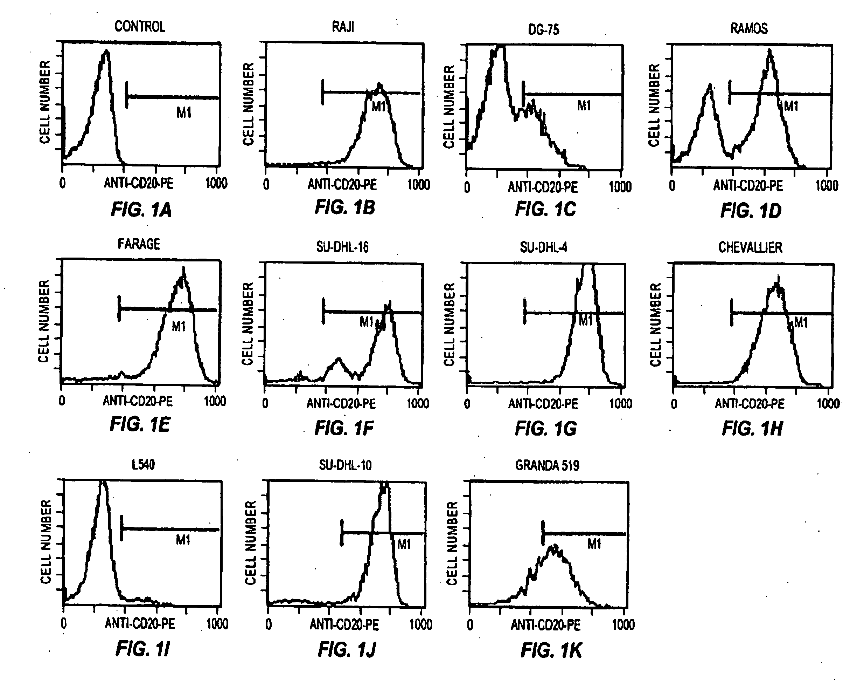

Hodgkin's and non-Hodgkin's lymphoma cell lines were analyzed for CD20 expression using FACs. The results of these studies are shown in FIG. 1A-1K. Cell lines with homogeneous high expression included Raji, Farage, SU-DHL-4, SU-DHL-16, Chevallier, and Granda 519. RAMOS, DG75, and SU-DHL-10 were found to have a bimodal expression level and the L540 Hodgkin's Disease cell line was negative for CD20 expression.

2. Effects of anti-HLA-DR Lym-1 on Raji lymphoma cell proliferation.

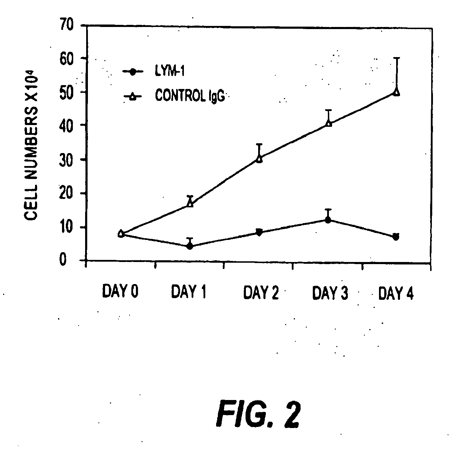

Raji cells (7.5x 10^{4/}/well) suspended in RPMI-1640 medium (Life Technologies, Inc., San Diego, CA) containing 10% characterized fetal calf serum (Hyclone Laboratories, Logan, UT) were cultured with 67µg/ml of Lym-1 or a negative control IgG (chTV-1). Cell proliferation was assessed at various time points using a hemocytometer and cell viability was determined by trypan blue dye exclusion. Media and antibody were renewed on day 3. As shown in FIG. 2, Lym-1 was found to inhibit Raji cell proliferation as compared with control antibody.

3. Apoptotic effects of Lym-1 and Rituximab on Raji cells with and without hypercross-linking.

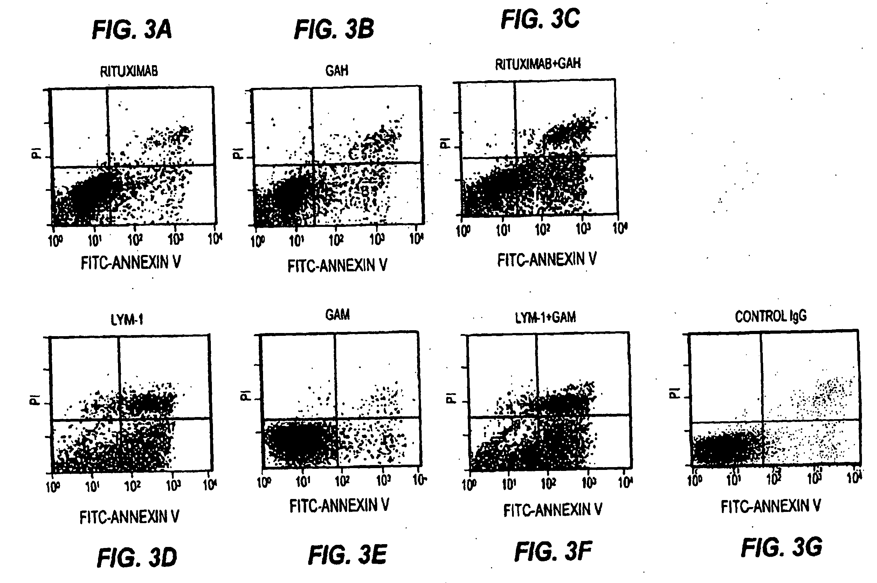

A. Annexin V/PI Staining. The ability of each MAb to induce apoptosis was assayed by Annexin V-FITC and PI counter-staining which detects phosphatidyl serine (PS) exposure on apoptotic cells and the loss of cell membrane integrity, respectively. As shown in FIG. 3A-3G, Lym-1 was able to induce apoptosis of Raji cells in a dose-dependent manner (data presented using single dose). Rituximab had little effect on the induction of apoptosis in these assays. However, hypercross-linking of Rituximab dramatically improved its ability to induce the apoptosis of Raji cells as shown in FIG. 3. Raji cells stained with control IgG is shown for comparison.

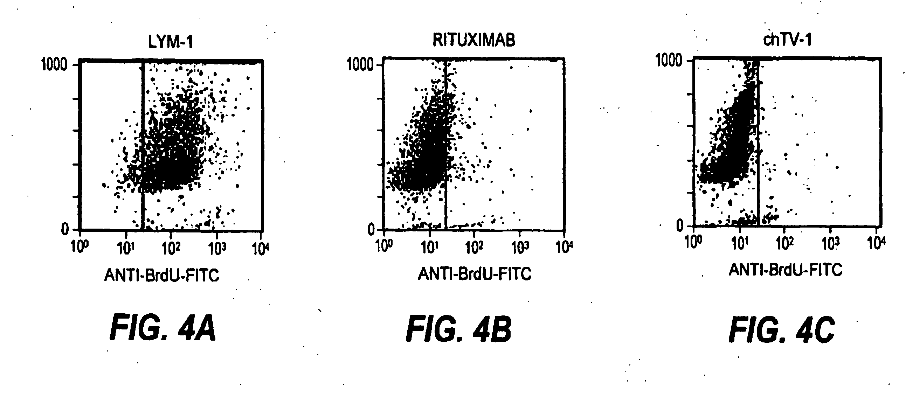

B. TUNEL Assay. As shown in FIG. 4A-4C, FACs analysis of the TUNEL assay showed extensive apoptosis induced by Lym-1 but not by Rituximab or chTv-1.

4. Induction of Apoptosis with Polymer Bound Antibodies

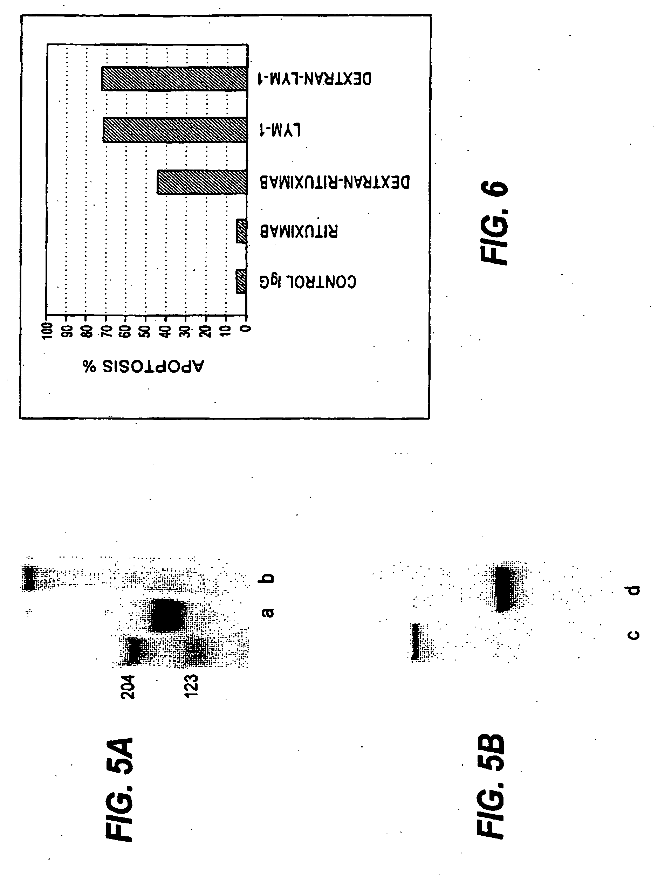

RITUXAN and Lym-1 were bound to dextran, a soluble, multi-branched, rigid polymer. For these studies, Dextran (200mg, average MW 10,000 Sigma-Aldrich) and sodium periodate (240mg, Sigma, Aldrich) were dissolved in 10ml of a 0.9% NaCl solution. After overnight incubation in the dark, the mixture was passed through a pre-equilibrated PD-10 column (Pharmacia) and eluted with 0.9% NaCl. The purified oxidized dextran was stored at 4°C in the dark. Concurrently, 100µl Lym-1 (23.6mg/ml), Rituximab (10mg/ml), and an irrelevant IgG (chTV-1, 11.8mg/ml) were dialyzed with sodium bicarbonate buffer (0.1M, pH 8.1) overnight. The dialyzed Lym-1, Rituximab, and chTV-1 were mixed with oxidized dextran at different molar ratios (1:1 and 1:2). After continuous shaking 6h in the dark, the reaction was reduced with PBS containing 5mg sodium borohydride (Sigma). Finally, each of the dextran-antibody conjugations was dialyzed with PBS overnight. The OD value of each conjugation was determined according to the antibody's concentration. The cross-linking efficiency was then determined by a non-reducing SDS gel (FIG. 5A-5B) which showed that after linkage to the dextran polymer, the molecular weights of Dextran-Lym-1, Dextran-Rituximab, and Dextran-chTV-1 (negative control) were markedly increased compared to unconjugated antibody.

Raji cells were incubated with increasing concentrations of reagent for 18h at 37°C in a 5% CO₂ incubator. Each sample was then stained with Annexin V/PI and subjected to FACs analysis as described above. The results of these studies for a single dose are shown below in FIG. 6. 73% apoptosis was achieved with Dextran-Lym-1 at the 667ng/ml concentration using a conjugation ratio of 1:1. These results are approximately the same level of apoptosis achieved with unconjugated murine Lym-1 seen in FIG. 3D-3F. Notably, 100% apoptosis was achieved when the concentration was increased to 6.7ug/ml (data not shown). By contrast, the Dextran-Rituximab preparations showed marked improvement in the induction of apoptosis from about 13% for unconjugated Rituximab (667ng/ml) to 30% for the Dextran-Rituximab at the same concentration (1:2 ratio of conjugation). When a higher concentration of Dextran-Rituximab was used (6.7ug/ml), the induction of apoptosis increased to 52%. In these experiments, the Dextran-chTV-1 negative control did not induce any significant level of apoptosis demonstrating that this phenomenon is antibody directed. These results demonstrate that anti-CD20 bound to a multi-branched rigid polymer such as dextran can directly induce apoptosis. Thus, polymer/antibody conjugates can simulate the cross-linking event produced by the addition of secondary GAM required for the induction of apoptosis by anti-CD20.

5. Biodistribution of Dextran-Rituximab in Raji lymphoma-bearing nude mice.

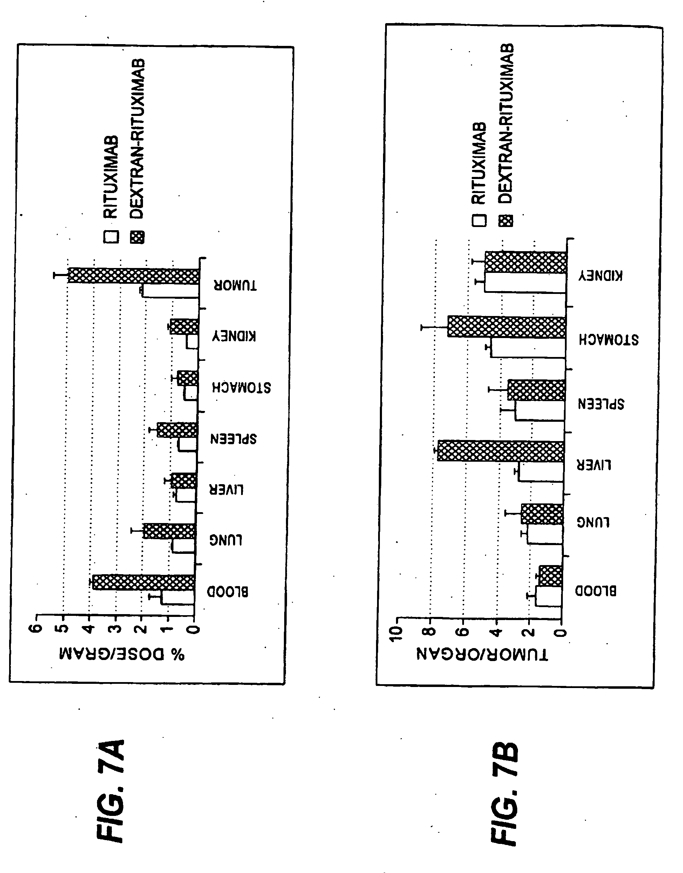

Biodistribution studies were performed in Raji lymphoma-bearing nude mice to measure the ability of the Dextran-Rituximab polymer to target tumor and clear from non-targeted normal tissues and organs. As shown in FIG. 7A, the Dextran-Rituximab showed enhanced uptake in tumor compared to equal doses of Rituximab alone. Remarkably, only a slight increase in uptake was seen in spleen and lung, two sites which have active reticuloendothelial systems. These data demonstrate that the Dextran-Rituximab polymer is pharmacologically viable *in vivo* and has improved tumor targeting characteristics in comparison to unconjugated Rituximab. Although not wishing to be bound by any theory, the enhanced uptake with the Dextran-Rituximab may be due to a longer serum half-life since the observed tumor/normal organ ratios are about the same for both reagents (FIG. 7B).

6. Biodistribution of Lym-1 and Dextran-Lym-1 in Raji lymphoma-bearing nude mice.

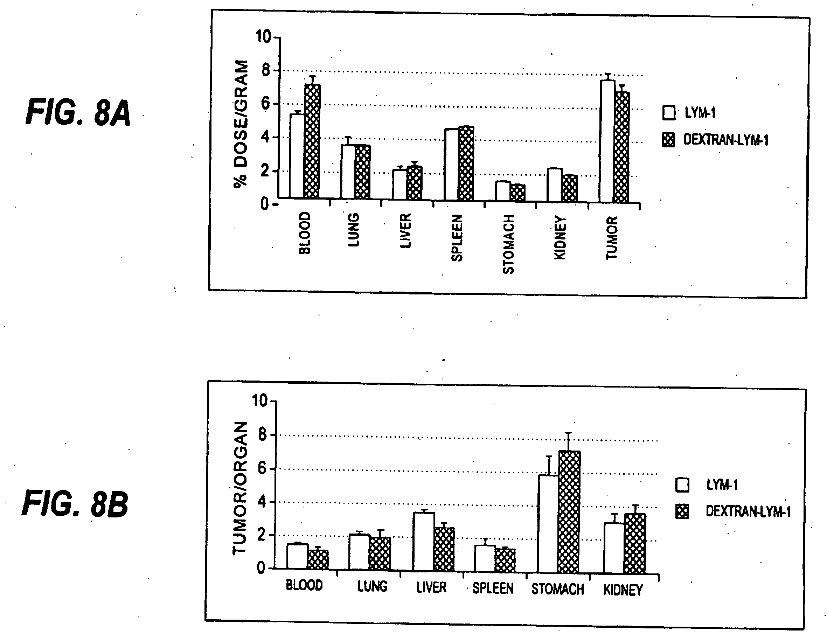

Comparative biodistribution studies were also performed with Lym-1 and Dextran-Lym-1. The 3 day biodistribution analysis shown in FIG. 8A-8B demonstrated little difference in the uptake or distribution of both reagents.

7. Enhancement of Therapeutic Efficacy by Dextran-RITUXAMAB in Raji lymphoma-bearing nude mice.

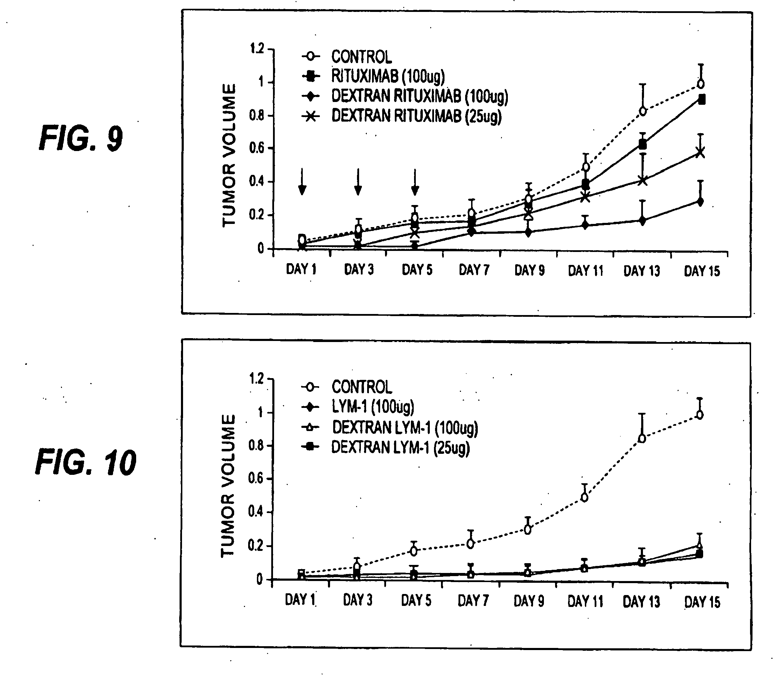

*In vivo* studies were performed to determine if enhancement of apoptosis induction by Dextran-Retuximab would also result in improved therapeutic effects on established Raji tumors heterotransplanted in nude mice. As shown in FIG. 9, a dose-dependent enhancement of clinical efficacy was clearly seen with Dextran-Rituximab in this tumor model using the described treatment regimen of 3 doses administered on alternate days. Tumor reduction was greater for Dextran-Rituximab than for monomeric Rituximab at similar protein doses.

8. Therapeutic efficacy of Lym-1 and Dextran-Lym-1 immunotherapy in Raji-bearing nude mice.

Comparative studies with Lym-1 and Dextran-Lym-1 shown in FIG. 10 Confirm that polymer based formulations did not improve the clinical efficacy of this antibody, which already showed outstanding induction of apoptosis by *in vitro* studies such as seen in FIG. 6.. Both native Lym-1 and polymer-Lym-1 produced a very effective tumor suppression compared to control treated mice.

Polymer bound anti-CD20 (Rituxamab) shows marked enhancement of the induction of early and late apoptosis in CD20 positive human lymphoma cell lines simulating the results seen with hypercross-linking methods. When tested *in vivo,* this formulation change enables anti-CD20 to be an effective reagent in a model in which ADCC is negligent due to the immunodeficiency status of nude mice. These results demonstrate that the induction of apoptosis can be a significant mechanism of tumor killing via CD20 if the antibody is presented as a multimer via a polymer formulation.

*Example 3. Antibody dimers prepared using homobifunctional and heterobifunctional cross-linking agents.*

*1. Homobifunctional cross-linkers.* These cross-linkers have two identical reactive groups (Table 2) and are used in a one-step reaction procedure to bind 2 MAb molecules (Park et al., J. Bio/. Chem. 261: 205-210, 1986; Browning, et al., J. Immunol. 143: 1859-1867, 1989). Homobifunctional cross-linkers include Sulfo-EGS and/or Sulfo-DST are used to bind two MAb molecules without disturbing antibody S-S bonds and without using harsh reducing agents. *Sulfo-DST* is a water-soluble analog useful for the preparation of homodimers in a one-step reaction. This cross-linker reacts with primary amino groups of MAbs via the N-hydroxysuccinimide (NHS) ester and forms a covalent amide bond. *Sulfo-EGS* is a water-soluble analog that is able to cross-link MAbs in a one-step reaction via primary amino groups by forming a covalent amide bond. As compared with DST, EGS has an extended spacer arm that reduces the effect of steric hindrance. All MAbs are cross-linked according to the manufacturer's instructions (Pierce) and according to an optimized procedure previously described (Khawli, et al., Cancer Biother & Radiopharm. 11: 203-215, 1996; Sharifi, et al., Q. J. Nucl. Med. 42: 242-249, 1998). After conjugation, the reaction mixture are dialyzed overnight and purified by chromatography on a Sephadex G-25. The different fractions are concentrated, filtered, and further analyzed by sodium dodecylsulfatepolyacrylamide gel electrophoresis (SDS-PAGE) and by high-pressure protein chromatography (HPLC) to determine purity and the number of antibody molecules per linker.

*2. Heterobifunctional cross-linkers.* These cross-linkers possess two different reactive groups that allow for sequential conjugations with specific groups of proteins (Table 3), minimizing undesirable polymerization or self-conjugation (Samoszuk, et al., Antibody, Immunoconj. & Radiopharm. 2: 37-45, 1989; Duncan, et al., Anal. Biochem. 132: 68-73, 1983). Two heterobifunctional cross-linkers such as Sulfo-SMCC and SATP are used sequentially to bind two MAbs through a stable thioether bond. Dimers are formed in a two-step reaction procedure. *a. SATP* is a reagent useful for introducing protected SH group into the MAbs and offers more steric freedom for the SH group. A stable covalent amide bond is formed from the reaction of the NHS ester with the primary amines of MAbs according to the manufacturer's instructions (Pierce). Deprotection to generate the SH bond is accomplished by deacetylation with hydroxylamine (without using reducing agents). *b. Sulfo-SMCC* is a water-soluble cross-linker analog and consists of an NHS ester and a maleimide group connected with a spacer arm which limits steric hindrance. NHS esters react with primary amines and maleimides react with SH groups. The NHS reaction is first performed according to the manufacturer's instructions (Pierce) and according to an optimized procedure as previously described (Khawli, et al., Cancer Biother & Radiopharm. 11: 203-215, 1996; Sharifi, et al., Q. J. Nucl. Med. 42: 242-249, 1998), then excess cross-linker is removed, followed by the addition of the component with the SH groups (MAb-SATP), and cross-linking is achieved by the formation of stable thioether linkages.

Both derivatized MAbs (MAb-SMCC and MAb-SATP) are mixed together and incubated at room temperature for 2-3 hours. The final mixture is dialyzed and purified by chromatography on a Sephadex G-25 column. The different fractions are concentrated, filtered, and further analyzed by SDS/PAGE and HPLC to determine purity and the number of antibody molecules per linker.

*Example 4. Preparation of Trimers by Using Trifunctional Cross-linkers.*

Trifunctional cross-linkers are a relatively new form of conjugation reagent, possessing three different reactive or complexing groups per molecule (Table 4). Cross-linkers such as Sulfo-TSAT and TMEA are used to bind three MAb molecules. 1. *Sulfo-TSAT* is a novel trifunctional cross-linking agent that is used for the preparation of trimers in a one-step reaction procedure. This cross-linker is a water-soluble analog which reacts with primary amino groups of MAbs via the N-hydroxysuccinimide (NHS) ester and forms a covalent amide bond. *2*. *TMEA* is a trifunctional cross-linking agent useful for the preparation of trimeric complexes of SH containing proteins in a two-step reaction. A heterobifunctional linker such as SATP is first used to introduce the SH group into the MAb (as described above), followed by the addition of TMEA. Cross-linking is achieved by the reaction of maleimides (present on TMEA) with the SH groups forming stable thioether linkages.

All trimeric antibodies are prepared according to the manufacturer's instructions (Pierce). After conjugation, the reaction mixture is dialyzed overnight and purified by chromatography on a Sephadex G-25. The different fractions are concentrated, filtered, and further analyzed by SDS/PAGE and HPLC as described above.

**Table 2. Homobifunctional Cross-linking Reagents (from commercial sources including Pierce, Sigma, and Fluka).**

| **Reagent** | **Chemical Name** |
|---|---|
| Sulfo-DST | Disulfosuccinimidyl tartrate |
| Sulfo-EGS | Ethylene glycol *bis*(sulfosuccinimidyl succinate) |
| BASED | *Bis*-[β-(4azidosalicylamido)ethyl]disulfide |
| BMB | 1,4-*Bis*-maleimidobutane |
| BMDB | 1,4 *Bis*-Maleimidyl-2,3-dihydroxybutane |
| BMH | *Bis*-Maleimidohexane |
| BMOE | *Bis*-Maleimidoethane |
| BM[PEO]₃ | 1,8-*Bis*-Maleimodotriethyleneglycol |
| BM[PEO]₄ | 1,11-*Bis-*Maleimodotetraethyleneglycol |
| BSOCOES/Sulfo-BSOCOES | *Bis*[2-(Sulfosuccinimidooxycarbonyloxy)-ethyl]sulfone |
| BS³ | *Bis*[sulfosuccinimidyl]suberate |
| DFDNB | 1,5-Difluoro-2,4-dinitrobenzene |
| DMA | Dimethyl adipimidate^{•}2HCl |
| DMP | Dimethyl pimelimidate^{•}2HCl |
| DMS | Dimethyl suberimidate^{•}2HCl |
| DPDPB | 1,4-Di-[3'(2'-pyridyldithio)propionamido]butane |
| DSG | Disuccinimidyl glutarate |
| DSP (Lomant's reagent) | Dithiobis[succinimidyl propionate] |
| DSS | Disuccinimidyl suberate |
| DST | Disuccinimidyl Tartrate |
| DTBP (Wang and Richard Reagent) | Dimethyl 3,3'-dithiobispropionimidate^{•}2HCl |
| DTME | Dithio-*bis*-maleimidoethane |
| DTSSP | 3,3'-Dithiobis[sulfosuccinimidyl propionate] |

**Table 3. Heterobifunctional Cross-linking Reagents (from commercial sources including Pierce, Sigma, and Fluka).**

| **Reagent** | **Chemical Name** |
|---|---|
| SAP | *N*-Succinimidyl-S-acetylthiopropionate |
| SATA | *N*-Succinimidyl S-acetylthioacetate |
| SMPT/Sulfo-LC-SMPT | Sulfosuccinimidyl-6-[α-methyl-α-(2-pyridyldithio)toluamido]hexanoate |
| SPDP/Sulfo-LC-SPDP | Sulfosuccinimidyl6-[3'-(2-pyridyldithio)-propionamido]hexanoate |
| SMCC/Sulfo-SMCC | Sulfosuccinimidyl 4-[*N-*maleimidomethyl]cyclohexane-1-carboxylate |
| EMCS/Sulfo-EMCS | *N-*[ε-Maleimidocaproyloxy]sulfosuccinimide ester |
| Sulfo-MBS | *m*-Maleimidobenzoyl-*N*-hydroxysulfosuccinimide ester |
| Sulfo-KMUS | *N*-[κ-Maleimidoundecanoyloxy]sulfosuccinimide ester |
| ABH | *p*-Azidobenzoyl Hydrazide |
| AEDP | 3-[(2-Aminoethyl)dithio]propionic acid^{•}HCl |
| AMAS | *N*-[α-Maleimidoacetoxy]succinimide ester |
| ANB-NOS | *N*-5-Azido-2-nitrobenzoyloxysuccinimide |
| APDP | *N*-[4-(*p*-azidosalicylamido)butyl-3'-(2'pyridyldithio)propionamide |
| APG | *p*-Azidophenyl glyoxyal monohydrate |
| ASBA | 4-[*p*-Azidosalicylamido]butylamine |
| BMPA | *N*-[β-Maleimidopropionic acid |
| BMPH | *N*-[β-Maleimidopropionic acid] Hydrazide^{•}TFA |
| BMPS | *N*-[β-Maleimidopropyloxy] succinimide ester |
| GMBS/Sulfo-GMBS | N-[γ-Maleimidobutyryloxy]sulfosuccinimide ester |
| HBVS | 1,6-Hexane-*bis*-vinylsulfone |
| KMUA | *N*-κ-Maleimidoundecanoic acid |
| M₂C₂H | 4-[*N*-Maleimidomethyl]cyclohexane-1-carboxylhydrazide^{•}HCl^{•} 1/2dioxane |
| MPBA | 3-Maleimidophenyl boronic acid |
| MPBH | 4-[4-*N*-Maleimidophenyl]butyric acid hydrazide•HCl |
| MSA | Methyl *N*-succinimidyl adipate |
| NHS-ASA | *N*-Hydroxysuccinimidyl-4-azidosalicylic acid |
| PDPH | 3-[2-Puridyldithio]propionyl hydrazide |
| PMPI | N-[*p*-Maleimidophenyl]isocyanate |
| SADP/Sulfo-SADP | Sulfosuccinimidyl[4-azidophenyldithio]propionate |
| SAED | Sulfosuccinimidyl 2-[7-azido-4-methylcoumarin-3-acetamido]ethyl-1,3'-dithiopropionate |
| SAND | Sulfosuccinimidyl 2-[*m*-azido-*o*-nitrobenzamido]ethyl-1,3'-dithiopropionate |
| SANPAH/Sulfo- | *N*-Sulfosuccinimidyl 6-[4'-azido-2'-nitrophenylamino]hexanoate |
| SANPAH | |
| SASD | Sulfosuccinimidyl-2[*p*-azidosalicylamido]ethyl-1-3'-dithiopropionate |
| SBAP | Succinimidyl 3-[bromoacetamido]propionate |
| SFAD | Succinimidyl-[perfluoroazidobenzamido]-ethyl-1,3'-dithiopropionate |
| SIA | *N*-Succinimidyl iodoacetate |
| SIAB/Sulfo-SIAB | Sulfosuccinimidyl[4-iodoacetyl]aminobenzoate |
| SMPB/Sulfo-SMPB | Sulfosuccinimidyl 4-[*p*-maleimidophenyl]butyrate |
| SMPH | Succinimidyl 4-[*p*-maleimidophenyl]butyrate |
| Sulfo-HSAB | *N*-Hydroxysulfosuccinimidyl-4-azidobenzoate |
| Sulfo-NHS-LC-ASA | Sulfosuccinimidyl[4-azidosalicylamido]hexanoate |
| SVSB | *N* Succinimidyl-[4-vinylsulfonyl]benzoate |
| TFCS | *N*-[ε-Trifluoroacetylcaproyloxy]succinimide ester |

**Table 4. Trifunctional Cross-linking Reagents (from commercial sources including Pierce, Sigma, and Fluka).**

| **Reagent** | **Chemical Name** |
|---|---|
| TSAT/Sulfo-TSAT | *Tri*-sulfosuccinimidyl aminotriacetate |
| TMEA | *Tris*-(2-maleimidoethyl)amine |
| Sulfo-SBED | Sulfosuccinimidyl [2-6-(biotinamido)-2-(p-azidobenzamido)-hexanoamido]ethyl-1-3'-dithiopropionate |
| THPP | β-[*Tris*(hydroxymethyl)phosphino]propionic acid (betaine) |

*Example 5*. *Generation of* Dynabeads^{®} M-280 *coated with Rituximab.*

Rituximab was coated on Dynabeads^{®} M-280 Tosylactivated (Dynal Biotech Inc., Brown Deer, WI) according to the manufacturer's protocol. Briefly, 7 × 10⁸ Dynabeads were washed twice with PBS and incubated with continuous rotation with 200µg of Rituximab or isotype control antibody in 500µl at 30°C for 18-20h. Dynabeads were subsequently pelleted using the magnetic bead attractor (Dynal Biotech Inc., Brown Deer, WI), and the unconjugated antibody in the supernatant was quantified by measuring OD at 280nm with a UV spectrophotometer (Ultraspec 2000, Pharmacia, Piscataway, NJ). The total amount of antibody conjugated to the Dynabeads was calculated by the equation (total antibody - unconjugated antibody = conjugated antibody on the beads). Dynabeads coated with antibodies were washed twice with PBS and 1 % (w/v) BSA, and free tosyl-groups were then treated with blocking solution (0.2M Tris-HCl pH 8.5; 0.1% BSA) at 37°C for 3-4h using end-to-end rotation, washed twice with PBS (0.1% BSA, 1% Tween-20), followed by another two washes with PBS (0.1% BSA). Finally, Dynabeads conjugated with antibodies were resuspended in PBS (0.05% sodium azide) and stored at 4°C. Sodium azide was removed prior to use by washing.

*Example 6. Generation of a Dextran-Rituximab Polymer* (1:2.4 Rituximab: oxidized dextran)

One hundred mg dextran (average MW 6,000 Fluka Chemie AG, Buchs, Switzerland) and 120mg sodium periodate (Sigma, St Louis, MO) were dissolved in 5mL of 0.9% NaCl solution and incubated for 18h in the dark at room temperature. The mixture was passed through a pre-equilibrated PD-10 column (Amersham Biosciences, Piscataway, NJ), eluted in 0.9% NaCl, and stored at 4°C in the dark.. Two mL Rituximab (10mg/mL) was passed through a pre-equilibrated PD-10 column and eluted with 0.1M sodium bicarbonate buffer (pH 8.1) (Sigma). The eluted Rituximab was diluted to 5mg in 1mL with 0.1M sodium bicarbonate buffer (pH 8.1), and mixed with 480µg oxidized dextran at a molar ratio of 1:2.4 (Rituximab: oxidized dextran). After continuous shaking for 6h in the dark, the reaction was reduced with 5mg sodium borohydride (Sigma) for 1h followed by dialysis in PBS. The dialysate was then passed through a 0.22 1µm non-pyrogenic syringe filter (Costar, Coming, NY), and a Pharmacia AKTA System (FPLC; (Amersham Pharmacia Biotech, Piscataway, NJ) was used to fractionate the dextran Rituximab mixture through a pre-equilibrated Superose 6 column in PBS at a flow rate of 0.5mL/min. Generally, the polymer had a retention time of approximately 16 min. The polymer fraction was further concentrated using a Centricon YM-100 (Millipore), sterilized by 0.22 µm filtration, and analyzed by SDS/PAGE. Proteins were analyzed under non-reducing conditions by SDS/PAGE on a 4% stacking, 5% separating gel. Protein bands were visualized by Coomassie blue staining.

*Example 7. Generation of a Rituximab Homodimer*

Two hetero-bifunctional cross-linkers, SATA (N-succinimidyl S-acethylthioacetate, (Pierce Biotechnology, Rockford, IL) and SMCC [succinimidyl 4-(maleimidomethyl) cyclohexane-1-carboxylate] (Pierce), were used to generate homodimers of Rituximab (28,29).

*1. Derivatization with SMCC.* Five mg of Rituximab at a concentration of 5mg/mL in 0.05M phosphate buffer containing 3mM Na₂EDTA (PBE; pH 7.5) and 2.5µL of SMCC (9.3mg/mL in dimethyl sulfoxide) were incubated with continuous rotation at room temperature for 1h using a SMCC/Rituximab molar ratio of 4.5. Conjugated protein was purified by chromatography on a PD-10 column in the same PBE buffer.

*2. Derivatization with SATA.* Five mg of Rituximab at a concentration of 5mg/mL in PBE buffer was mixed with 2.5µL of SATA (5.8mg/mL in dimethyl sulfoxide) and incubated at room temperature for 1h using a SATA/Rituximab molar ratio of 4.0. The excess SATA was then removed by chromatography on a PD-10 column and the purified protein was deacetylated with 5mg of hydroxylamine-HCl (Sigma) for 5 min at room temperature. Excess hydroxylamine-HCl was removed by chromatography on a PD-10. column.

3. Rituximab Homodimer Formation and Physical Characterization

SMCC- and SATA-derived proteins were mixed together and incubated with continuous rotation at room temperature for 1-2h. The preparation was dialyzed overnight in PBS at 4°C, sterilized by filtration through a 0.22µm non-pyrogenic filter (Costar, Coming, NY), and further fractionated on a Superose 6 Column (FPLC) using the Pharmacia AKTA described above at flow rate of 0.5mL/min.

In general, the polymer revealed a retention time of approximately 16 min while the dimer revealed a retention time of approximately 29 min by FPLC. The protein fractions from FPLC were further concentrated using a Centricon YM-100 (Millipore, Billerica, MA), filter sterilized, and analyzed by SDS/PAGE. The expected molecular weights of the Rituximab dimer was observed for the dimer preparation by non-reducing SDS-PAGE. A 2% agarose gel was used with murine IgM as a standard (970 kDa) for sizing the polymer. By this method, one band was resolved for the Rituximab polymer which was slightly higher than that of IgM corresponding to the molecular weight of approximately 5 immunoglobulin molecules.

*Example 8. Results Comparing Rituximab Dimer with Rituximab Polymer* (1:2.4 Rituximab: oxidized dextran)

1. Avidity Studies of Rituximab Preparations.

Avidity binding studies were conducted in which ¹²⁵I-labeled Rituximab preparations were incubated with Raji lymphoma cells and the bound radioactivity was used to calculate the avidity constant (Ka) by Scatchard analysis. Using this method, the Rituximab polymer was found to have an avidity constant of 3.16 x 10⁸ M⁻¹, while the monomer and dimer had avidity constants of 3.6 x 10⁸ M⁻¹ and 2.09 x 10⁸ M⁻¹, respectively. These studies demonstrate that the Rituximab polymer and dimer have similar binding avidities to antigen as Rituximab itself, facilitating subsequent direct comparisons in vitro and in vivo.

2. Induction of Apoptosis by Rituximab Preparations

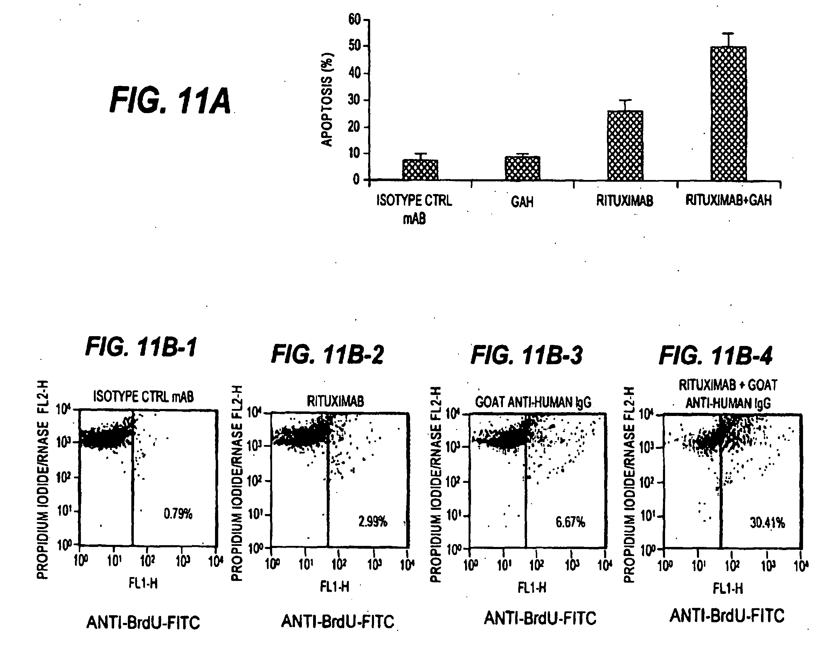

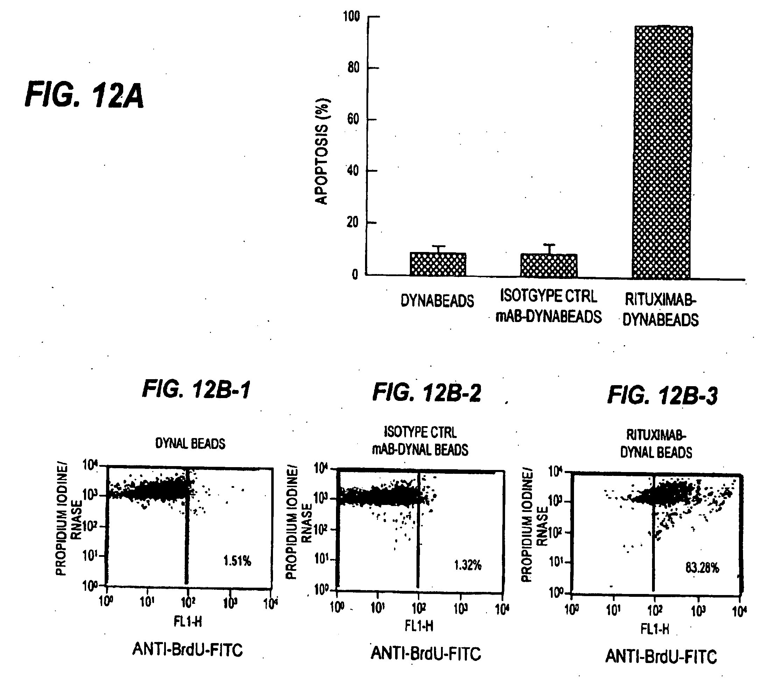

Raji lymphoma cells were treated with Rituximab alone or hyper-crosslinked Rituximab for 18-20h at 37°C. The amount of apoptosis was measured by Annexin V/ PI staining (FIG. 11A) and by the TUNEL assay (FIG. 11B1-B4). In this experiment, rituximab monomer induced only a minimal level of apoptosis in Raji cells, about 26% in the Annexin assay and 2.99% in the TUNEL assay. The background apoptosis for rituximab monomer in the Annexin assay in FIG. 11B1-4 is higher than that shown in FIG. 6, the difference being ' possibly due to assay variation over time. By comparison, GAH hyper-crosslinked Rituximab caused a higher level of apoptosis (about 50% in the Annexin assay and 30.41% in the TUNEL assay; FIG. 11A and 11B-14). Rituximab conjugated to Tosyl-activated Dynabeads (2.8µm in diameter) induced the best apoptosis levels detected by both Annexin V/PI staining and TUNEL assays (about 96% and 83.28%, respectively; FIG. 12A and 12B1-B3).

Apoptosis assays were used to analyze the ability of the Rituximab polymer and dimer to induce apoptosis. TUNEL assays showed that the Rituximab polymer induced 57.8% apoptosis while the dimer produced only 2.99% (results not shown).

The apoptosis inducing quality of various Rituximab preparations was tested on 10 different cell lines including 10 that were CD20⁺ and 2 that were CD20 The results are shown in Table 5.

**TABLE 5. Induction of Apoptosis in CD20 Positive and Negative Cell Lines by Rituxamab Monomer, Dimer, and Polymer Preparations as measured by Annexin V.**

| **Lymphoma Cell Lines CD20⁺** | **CD20 Expression (MFI¹)** | **Untreated** | **Rituximab Monomer** | **Rituximab Dimer** | **Rituximab Polymer** |
|---|---|---|---|---|---|
| Raji | 514.34 | 4.56² | 27.86 | 26.97 | 66.07 |
| B35M | 349.33 | 6.02 | 13.13 | 8.92 | 56.36 |
| RAMOS | 374.44 | 5.04 | 7.66 | 6.08 | 24.68 |
| SU-DHL-4 | 672.19 | 4.78 | 10.67 | 7.31 | 11.54 |
| SU-DHL-6 | 685.64 | 28.16 | 45.36 | 42.50 | 55.89 |
| SU-DHL-10 | 631.97 | 7.01 | 27.85 | 25.69 | 36.88 |
| SU-DHL-16 | 585.14 | 15.07 | 27.28 | 23.06 | 31.43 |
| Farage | 643.63 | 19.22 | 21.25 | 20.56 | 46.11 |
| Granda 519 | 495.48 | 11.78 | 20.27 | 18.61 | 48.30 |
| Chevallier | 408.99 | 18.18 | 18.80 | 16.80 | 40.70 |
| CD20⁻ | | | | | |
| DG-75 | 180.78 | 6.82 | 8.04 | 6.85 | 7.85 |
| L540 | 196.37 | 16.23 | 16.54 | 16.02 | 16.00 |

| | | | | | |
|---|---|---|---|---|---|
| ¹ Mean fluorescence intensity. ² Percent non-viable cells. | | | | | |

In the CD20⁺ cell lines (Raji, B35M, Ramos, Farage, Granda 519, and Chevallier), apoptosis was significantly increased in the cells treated by Rituximab polymer compared to those treated with the dimer or monomer. However, not all CD20⁺ cells treated by Rituximab polymershowed significant apoptosis. It is believed that some of these lymphoma cell lines may have different intracellular pathways associating with CD20 ligation. No significant apoptosis was detected in any of the CD20⁻ cell lines treated with the different Rituximab preparations, likely due to the lack of antibody binding.

3. Immunofluorescence Microscopy Studies.

Rituximab monomer, dimer, and polymer were incubated with Raji cells and then fixed with 2% paraformaldehyde before being stained with secondary FITC-conjugated goat anti-mouse F(ab')₂. In addition, the effects of hyper-cross-linking were studied by treating cells tandomly with Rituximab and FITC-conjugated goat anti-mouse F(ab')₂ before being fixed with 2% paraformaldehyde. The results demonstrate that Rituximab monomer and dimer treatments produced a ring pattern of immunofluorescence, indicating that CD20 is evenly distribution on the surface of the lymphoma cells. By contrast, cells treated with Rituximab polymer or Rituximab + secondary antibody were found to have a more segregated polar staining pattern consistent with the movement and fixation of CD20 into lipid rafts. These results indicate that CD20 clustering and fixation in lipid rafts is consistent with apoptosis induction.

4. Caspase 3 Activation Studies.

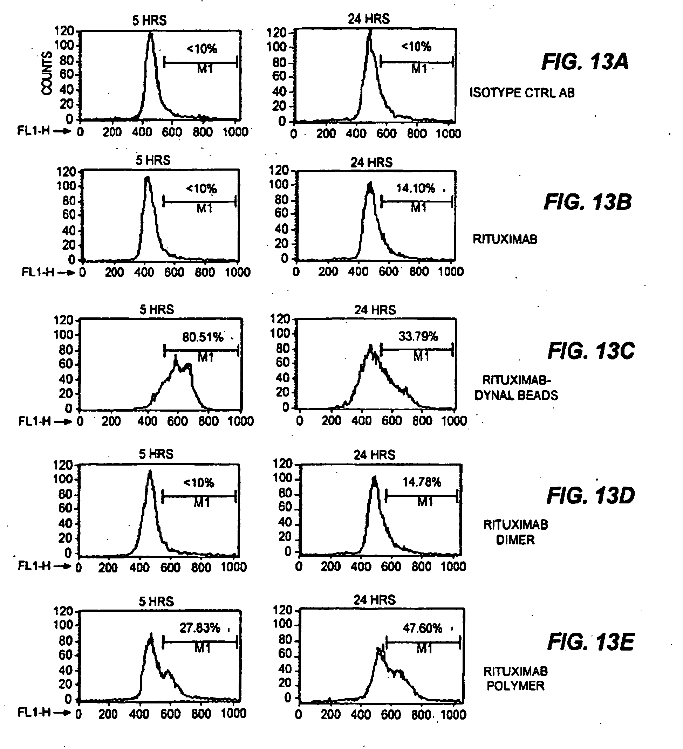

As shown in FIG. 13A-13E, Raji lymphoma cells treated with Rituximab monomer or dimer were found to have basal levels of Caspase 3 activity similar to controls. By contrast, Raji lymphoma cells treated with Rituximab polymer or Rituximab coated Dynabeads showed significant Caspase 3 activation. For polymer treated cells, Caspase 3 activity started to elevate at 5h after treatment and reached a peak at 24h. For those cells treated with Rituximab coated Dynabeads, Caspase 3 activity peaked at 5h after treatment and declined at 24h. From these experiments, it appears that early induction of apoptosis correlated with a more vigorous activation of Caspase 3 as seen with the samples treated with Rituximab coated Dynabeads.

5. Pharmacokinetic and Biodistribution Studies.

Whole-body radioactivity studies in BALB/c mice were performed to establish differences in pharmacokinetic characteristics between the ¹²⁵I-labeled Rituximab monomer, dimer, and polymer. From these studies, it was determined that the ¹²⁵I-Rituximab has a T_{1/2} of 96h (p< 0.01) while the dimer and polymer showed slower total whole-body clearance varying of 120h and 144h, respectively (p<0.01). The extended clearance related to the increase in molecular weight.

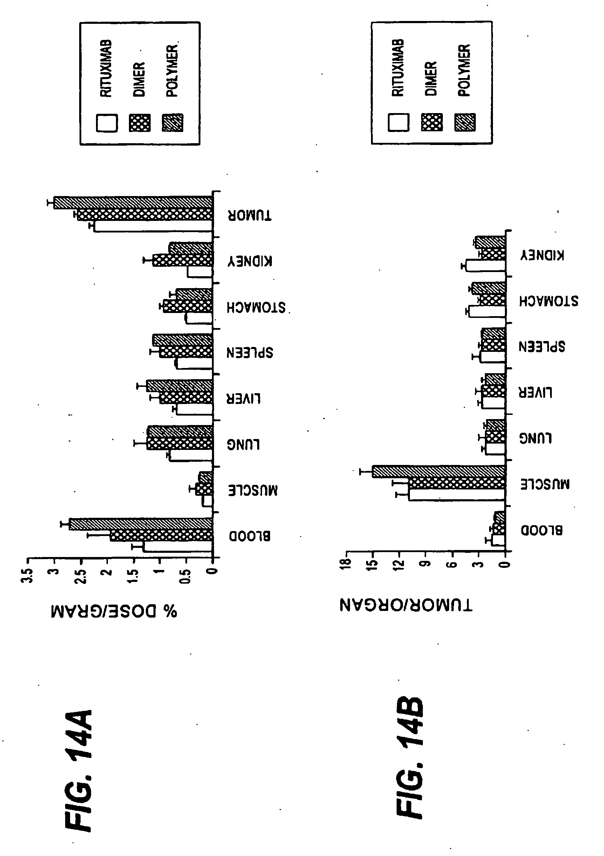

For the biodistribution studies, groups of Raji-bearing nude mice were injected intravenously either with ¹²⁵I-labeled Rituximab monomer, dimer, or polymer. Three days later, the mice were sacrificed and the radioactivity in each organ was measured to calculate the percentage injected dose/gram (% ID/g) and tumor/organ ratios (cpm per gram tumor/cpm per gram organ). As shown in FIG. 14A, all three Rituximab preparations effectively localized to the Raji human lymphoma xenografts resulting in approximately similar tumor-to-organ ratios (FIG. 14B). The data further illustrate the specificity of tumor targeting with all three Rituximab preparations as indicated by the high tumor uptake observed.

6. Immunotherapy Studies.

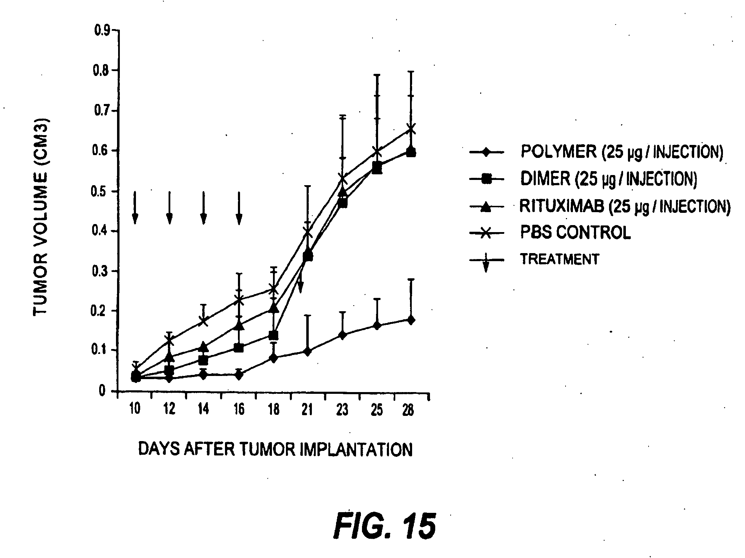

As shown in FIG. 15, treatment of established Raj i xenografts did not commence until day 10 when the tumors were palpable in size. In those groups of mice receiving the Rituximab monomer or dimer, tumor growth was either unaffected or slightly slower than that seen in the untreated control group. By contrast, mice receiving the Rituximab polymer demonstrated marked tumor regression. The average tumor volume of the polymer-treated group on the 28^{th} day after tumor implantation were <30% of the tumor volume in the PBS-treated control group (p < 0.01). These findings are particularly notable in view of the fact that all these preparations had comparable avidity constants and biodistribution characteristics in this same tumor model.

7. Conclusions.

Direct induction of apoptosis by Rituximab is very ineffective unless the antibody is hyper-crosslinked by secondary antibody or immobilized on plastic (5-7 and 19). A polymer of Rituximab that consisted of at least 5 immunoglobulins per dextran molecule demonstrated potency *in vitro* and *in vivo* as compared with Rituximab monomer or dimer. The polymer induces apoptosis of CD20⁺ human lymphoma cell lines directly upon binding, in contrast to the monomer or dimer preparations. In addition, the Rituximab polymer was much more effective *in vivo* against implanted Raji lymphomas using dosages in which the monomer and dimer preparations had little effect. Notably, all three antibody preparations have essentially equal avidity for CD20 and show similar pharmacokinetic properties and biodistribution characteristics in tumor-bearing nude mice.

Rituximab coated Dynabeads induced maximum levels of apoptosis in Raji cells in which >90% of cells were shown to become apoptotic. Dynabead presentation may be analogous to FcR-bearing cells *in vivo.* Antibody coated Dynabeads provides a simple method to identifying antibodies in which apoptosis induction via FcR-bearing cell presentation is an important mechanism of action.

Immunofluorescence microscopy showed that CD20 antigen clustering is associated with superior induction of apoptosis with Rituximab antibody formulations. Thus, CD20 antigen clustering detected by immunofluorescence microscopy can be used to predict antibody formulations that can induce apoptosis.

Several mechanisms have been proposed to explain the effectiveness of Rituximab treatment both in animal models and man. The notion that ADCC plays a major role *in vivo* is the currently prevailing hypothesis and several publications have addressed the importance of antibody presentation via FcRs (20, 59, 60). In mouse models, FcγRII has an inhibitory role in Rituximab's therapeutic effect (59), but some studies produced contradictory results (61). Specifically, mouse fibroblast Ltk⁻ cells that were transfected with FcγRII still facilitated signaling induced apoptosis (6). In the results herein, the effectiveness of the Rituximab polymer preparation in suppressing Raji lymphoma *growth in vivo* demonstrates that apoptosis is a powerful mechanism for treating lymphomas. Caspase 3 induction was seen with Rituximab coated Dynabeads and polymer while only basal levels of Caspase activity was observed with Rituximab monomer and dimer. Higher and more efficient apoptosis induction correlated with earlier and more active increases in Caspase 3.

In summary, effective therapy of lymphoma via induction of apoptosis was achieved using multimeric forms of anti-CD20 antibody beyond those of dimeric antibody preparations. *In vitro* screening of suitable apoptotic inducing anti-CD20 formulations can be tested using secondary antibodies, FcR bearing cells, or Rituximab coated Dynabeads. *In vivo,* hyper-cross-linking appears to require presentation by FcR bearing cells (59). However, anti-CD20 polymer preparations prepared as described herein appear to obviate the need for effector cells. Thus, anti-CD20 polymers may be especially useful in lymphoma patients who have the low FcR phenotype and who are non-responsive to Rituximab monotherapy (62).

**REFERENCES**
1. Chang, KL, Arber, DA, and Weiss, LM. CD20: A review. Applied Immunohistochem. 4:1-15, 1996.
2. Stashenko, P., Nadler, LM, Hardy, R., and Schlossman, SF. Characterization of a human B lymphocyte-specific antigen. J. Immunol. 125:1678-1685, 1980.
3. Enfeld, DA, Brown, JP, Valentine, MA, Clark, EA, and Ledbetter, JA. Molecular cloning of the human B cell CD20 receptor predicts a hydrophobic protein with multiple transmembrane domains. EMBO J. 7:711-717, 1988.
4. Tedder, TF and Engel, P. CD20: A regulator of cell-cycle progression of B lymphocytes. Immunol. Today 15:450-454, 1994.
5. Press, OW, Farr, AG, Borroz, KI, Anderson, SK, and Martin, PL. Endocytosis and degradation of monoclonal antibodies targeting human B-cell malignancies. Cancer Res. 49:4906-4912, 1989.
6. Hainsworth, John D., Burris, Howard A., Morrissey, Lisa H., Litchy, Sharlene, Scullin, Daniel C., Bearden, James D., Richards, Paul, and Greco, F. Anthony. Rituximab monoclonal antibody as initial systemic therapy for patients with low-grade non-Hodgkin lymphoma. Blood 95:3052-3056, 2000.
7. Hofmeister, Joseph K., Cooney, Damon, and Coggeshall, K. Mark. Clustered CD20 induced apoptosis: Src-family kinase, the proximal regulator of tyrosine phosphorylation, calcium influx, and caspase 3-dependent apoptosis. Blood Cells, Molecules, and Diseases 26:133-143, 2000.
8. Shan, Daming, Ledbetter, Jeffrey A., and Press, Oliver W. Signaling events involved in anti-CD20-induced apoptosis of malignant human B cells. Cancer Immunol. Immunother. 48:673-683, 2000.
9. Shan, Daming, Ledbetter, Jeffrey A., and Press, Oliver W. Apoptosis of malignant human B cells by ligation of CD20 with monoclonal antibodies. Blood 91:1644-1652, 1998;
10. Clark, EA, Shu, G, and Ledbetter, JA. Role of the Bp35 cell surface polypeptide in human B-cell activation. Proc. Natl. Acad. Sci. (USA) 82:1766-1770, 1985.
11. Deans, Juli P., Robbins, Stephen M., Polyak, Maria J., and Savage, Janice A. Rapid redistribution of CD20 to a low density detergent-insoluble membrane compartment. J. Biol. Chem. 273:344-348, 1998.
12. Polyak, Maria J., Tailor, Sweta H., and Deans, Julie P. Identification of a cytoplasmic region of CD20 required for its redistribution to a detergent-insoluble membrane compartment. J. Immunology 161:3242-3248, 1998.
13. Semac, Isabelle, Palomba, Carmen, Kulangara, Karina, Klages, Natacha, Van Echten-Deckert, Gerhild, Borisch, Bettina, and Hoessli, Daniel C. Anti-CD20 therapeutic antibody Rituximab modifies the functional organization of rafts/microdomains of B lymphoma cells. Cancer Res. 63:534-540, 2003.
14. Craig, Mark S., Morgan, Suzanne M, Chan HT Claude, Morgan, B. P., Filartov, A.V., Johnson, Peter, French, Ruth R., and Glennie, Martin J. Complement-mediated lysis by anti-CD20 mAb correlates with segregation into lipid rafts. Blood 101:1045-1052, 2003.
15. Harjunpaa, A., Junnikkala, S, and Meri, S. Rituximab (anti-CD20) therapy of B-cell lymphomas: Direct complement killing is superior to cellular effector mechanisms. Scand. J. Immunology 51:634-641, 2000.
16. Manches, Olivier, Lui, Gabrielle, Chaperot, Laurence, Gressin, Remy, Molens, Jean-Paul, Jacob, Marie-Christine, Sotto, Jean-Jacques, Leroux, Dominique, Bensa, Jean-Claude, and Plumas, Joel. In vitro mechanism of action of Rituximab on primary non-Hodgkin lymphomas. Blood 101:949-954,2003.
17. Weng, Wen-Kai and Levy, Ronald. Expression of complement inhibitors CD46, CD55, and CD59 on tumor cells does not predict clinical outcome after Rituximab treatment in follicular non-Hodgkin lymphoma. Blood 98:1352-1357, 2001.
18. Maloney, DG, Smith, B, and Appelbaum, FR. The anti-tumor effect of monoclonal antibody anti-CD20 antibody (mAb) therapy includes direct anti-proliferative activity and induction of apoptosis in CD20 positive non-Hodgkin's lymphoma (NHL) cell lines. Blood 88:673a, 1996.
19. Van der Kolk, LE, Evers, LM, Omene, C, Lens, SMA, Lederman, S, van Lier, RAW, van Oers, MHJ, and Eldering, E. CD20-induced B cell death can bypass mitochondria and caspase activation. Leukemia 16:1735-1744, 2002.
20. Cartron, Guillaume, Dacheux, Laurent, Salles, Gilles, Solat-Celigny, Philippe, Bardos, Pierre, Colombat, Philippe, and Watier, Herve. Therapeutic activity of humanized anti-CD20 monoclonal antibody and polymorphism in IgG Fc receptor FcγRIIIa gene. Blood 99:754-758, 2002.
21. Craig, M.S. Zhang, L, French, R.R. and Glennie, M.J. Analysis of the interaction of monoclonal antibodies with surface IgM on neoplastic B-cells. Br. J. Cancer 79:850-857. 1999.
22. Vitetta, ES and Uhr, J. Monoclonal antibodies as agents: An expanded role for their use in cancer therapy. Cancer Res. 54:5301-5309, 1994.
23. Levy, Ronald and Miller, Richard A. Therapy of lymphoma directed at idiotypes. J. Natl. Cancer Inst. Monogr. 10:61-68, 1990.
24. Ghettie, MA, Picker, LJ, Richardson, JA, Tucker, K, Uhr, JW and Vitetta, ES. Anti-CD19 inhibits the growth of human B-cell tumor lines in vitro and of Daudi cells in SCID mice by producing cell cycle arrest. Blood 83:1329-1336, 1994.
25. Bridges, SH, Druisbeek, AM, and Longo, DL. Selective in vivo antitumor effects of monoclonal anti-IA antibody on B-cell lymphoma. J. Immunol. 139:4242-4249, 1987.
26. Chaouchi, N, Vazques, A, Galanaud, P, and Leprince, C. B cell antigen receptor-mediated apoptosis: Importance of accessory molecules CD19 and CD22, and of surface IgM cross-linking. J. Immunol. 154:3096-3104, 1995.
27. Kerr, JF, Wyllie, AH, and Currie, AR. Apoptosis: A basic biological phenomenon with wide-ranging implications in tissue kinetics. Br. J. Cancer 26:239-257, 1972.
28. Ghetie, Maria-Ana, Podar, Erika M., Ilgen, Amy, Gordon, Brian E., Uhr, Jonathan W., and Vitetta, Ellen S. Homodimerization of tumor-reactive monoclonal antibodies markedly increases their ability to induce growth arrest or apoptosis of tumor cells. Proc. Natl. Acad. Sci. (USA) 94:7509-7514, 997.
29. Ghetie, Maria-Ana, Bright, Helen, and Vitetta, Ellen S. Homodimers but not monomers ofRituxan (chimeric anti-CD20) induce apoptosis in human B-lymphoma cells and synergize with a chemotherapeutic agent and an immunotoxin. Blood 97:1392-1398, 2001.
30. Zhao, Yunfeng, Lou, Dingyuan, Burke, John, and Kohler, Heinz. Enhanced anti-B cell tumor effects with anti-CD20 superantibody. J. Immunotherapy 25:57-62, 2002.
31. Epstein, Alan L., Marder, Robert J., Winter, Jane N., Stathopoulos, Efstathios, Chen, Feng-Ming, Parker, John W., and Taylor, Clive R.: Two new monoclonal antibodies, Lym-1 and Lym-2, reactive with human B- lymphocytes and derived tumors with immunodiagnostic and immunotherapeutic potential. Cancer Res. 47:830-840, 1987.
32. Rose, Larry M., Gunasekera, Angelo H., DeNardo, Sally J., DeNardo, Gerald L., and Meares, Claude E. Lymphoma-selective antibody Lym-1 recognizes a discontinuous epitope on the light chain of HLA-DR10. Cancer Immunol. Immunother. 43:26-30, 1996.
33. Rose, L.M., Deng, C.T., Scott, S.L., Xiong, C.Y., Lamborn, K.R., Gumerlock, P.H., DeNardo, G.L., and Meares, C.F. Critical Lym-1 binding residues on polymorphic HLA-DR molecules. Mol. Immunol. 36:789-797, 1999.
34. DeNardo, S.J., DeNardo, G.L., O'Grady, L.F., Hu, E., Sytsma, V.M., Mills, S.L., Levy, N.B., Macey, D.J., Miler, C.H., and Epstein, A.L.: Treatment of B cell malignancies with 131I Lym-1 monoclonal antibodies. Int. J. Cancer 3:96-101, 1988.
35. DeNardo, Gerald L, DeNardo, Sally J, O'Grady, Lois F, Levy, Norman B, Adams, Gregory P., and Mills, Stanley. Fractionated radioimmunotherapy of B-cell malignancies with 131-1-Lym-1. Cancer Res. 50:1014s-1016s, 1990.
36. DeNardo, Gerald L, DeNardo, Sally J, Goldstein, Desiree S, Droger, Linda A, Lambom, Kathleen R,. Levy, Norman B, McGahan, John P, Salado, Qansy, Shen, Sui, and Lewis, Jerry P. Maximum-tolerated dose, toxicity, and efficacy of 131-I-Lym-1 antibody for fractionated radioimmunotherapy of non-Hodgkin's lymphoma. J. Clin. Oncol. 16:3246-3256, 1998.
37. DeNardo, Gerald L, O'Donnell, Robert T., Rose, Larry M., Mirick, Gary R., Kroger, Linda A., and DeNardo, Sally J. Milestones in the development of Lym-1 therapy. Hybridoma 18:1-11, 1999.
38. Ottonello, L., Epstein, A.L., Dapino, P., Barbers, P., Morone, P., and Dallegri, F.: Monoclonal Lym-1 antibody-dependent cytolysis by neutrophils exposed to GM-CSF. Intervention of FCγRII (CD32), CAD11b,-CD18 integrins and CD66b glycoproteins. Blood 93:3505-3511, 1999.
39. Ottonello, Luciano, Epstein, Alan L., Dapino, Patrizia, Barbera, P., Morone, P., and Dallegri, Franco: Monoclonal Lym-1 antibody-targeted lysis of B. lymphoma cells by neutrophils exposed to Granulocyte-Macrophage Colony-Stimulating Factor: Intervention of FcγRII (CD32), CD11b-CD18 integrins, and CD66b glycoproteins. Blood 93:3505-3511, 1999.
40. Ottonello, Luciano, Epstein, Alan L., Mancini, Marina, Tortolina, Giuseppe, Dapino, Patrizia, and Dallegri, Franco: Monoclonal Lym-1 antibody-targeted lysis of B lymphoma cells by neutrophils. Evidence for two mechanisms of FcγRII-dependent cytolysis. J. Leukocyte Biology 68:662-668, 2000.
41. Hu, Peisheng, Glasky, Michelle S., Yun, Aoyun, Alauddin, Mian M., Hornick, Jason L., Khawli, Leslie A., and Epstein, Alan L.: A human-mouse chimeric Lym-1 monoclonal antibody with specificity for human lymphomas expressed in a baculovirus system. Human Antibod. Hybridomas 6:57-67,1995.
42. Pulvertaft RJV. Cytology of Burkitt's tumour (African lymphoma). Lancet 1: 238-240, 1964.
43. Epstein, Alan L., Levy, Ronald, Kim, Hun, Henle, Werner, Henle, Gertrude, and Kaplan, Henry S.: Biology of the human malignant lymphomas: IV. Functional characterization of ten diffuse histiocytic lymphoma cell lines. Cancer 42:2379- 2391, 1978.
44. Epstein, Alan L., Variakojis, Daina, Berger, Carol, and Hecht, Barbara K.: Use of novel chemical supplements in the establishment of three human malignant lymphoma cell lines (NU-DHL-1, NU-DUL-1, NU-AmB-1) with chromosome 14 translocations. Int J Cancer 35:619-627, 1985.
45. Zegers, N, Gerritse, CD, Deen, C, Boersma W, and Claassen E: An improved conjugation method for controlled covalent coupling of synthetic peptides to proteins using glutaraldehyde in a dialysis method. J. Immunol. Methods. 130: 195-200, 1990
46. Lutsenko, SV, Feldman, NB, and Severin, SE: Cytotoxic and antitumor activities of doxorubicin conjugates with the epidermal growth factor and its receptor-binding fragment. J. Drug Targeting. 10: 567-571, 2002.
47. Chen, F-M, LeBerthon, B,Naeve, GS, and Epstein, AL: Daunomycin and doxorubicin Lym-1-drug conjugates for the treatment of malignant lymphomas. In: Cancer Chemotherapy: Concepts, Clinical Investigations and Therapeutic Advances (FM Muggia, Ed.), Kluwer Publishers, Boston, pp.97-104, 1988,
48. Hurwitz, E, Maron, R, Arnon, R, Wilcheck, M, and Sela, M: Daunomycinimmunoglobulin conjugates uptake and activity in vitro. Eur. J. Cancer 14: 1213-1220, 1978.
49. Hurwitz, E, Levy, R, Maron, R, Wilcheck, M, Arnon, R, and Sela, M: The covalent binding of daunomycin and adriamycin to antibodies, with retention of both drug and antibody activities. Cancer Res. 35: 1175- 1181, 1975.
50. Tomalia, DA, Naylor, AM and Goddard III, WA: Starburst dendrimers: Molecular level control of size, shape, surface chemistry; topology, and flexibility from atoms to macroscopic matter. Angnew Chem. Int. Ed. Engl. 29: 138-175, 1990.
51. Wu, C, Brechbiel, MW, Kozak, RW, and Gansow, OA: Metal-chelate-dendrimer-antibody constructs for use in immunotherapy and imaging. Bioorg. Med. Chem. Lett. 4: 449-454, 1994.
52. Kobayashi, H, Sato, N, Saga, T, Nakamoto, Y, et al, Monoclonal antibody dendrimer conjugates enable radiolabeling of antibody with markedly high specific activity with minimal loss of immunoreactivity. Eur. J. Nucl. Med. 27: 1334-1339,2000.
53. Marrieda, M, Saga, T, Kobayashi, H, Ishimori, T, Higashi, T, Sato, N, Brechbiel, MW, and Konishi, J: Radiolabeling od f Avidin with very high Specific activity for internal radiation therapy of intraperitoneally disseminated tumors. Clin. Cancer Res. 9: 3756-3762, 2003.
54. Abu-Rmaileh, R, Attwood D, D'Emanuelle, A: Dendrimers in cancer Therapy. Drug Delivery Systems and Sciences. 3: 65-70, 2003.
55. Frankel, M. E. and Gerhard, W. The rapid determination of binding constants for antiviral antibodies by a radioimmunoassay: an analysis of the interaction between hybridoma proteins and influenza virus. Mol. Immunol. 16: 101-106, 1979:
56. Hornick, J. L., Sharifi, J. Khawli, L. A., Hu, P., Biela, B. H., Mizokami, M. M., Yun, A., Taylor, C. R., and Epstein, A. L., A new chemically modified chimeric TNT-3 monoclonal antibody directed against DNA for the radioimmunotherapy of solid tumors. Cancer Biother. & Radiopharm.. 13: 255-68, 1998.
57. Sharifi J, Khawli LA, Hu P, King S, and Epstein AL. Characterization of a phage display derived human monoclonal antibody (NHS76) counterpart to chimeric TNT-1 directed against necrotic regions of solid tumors. Hybridoma & Hybridomics 20: 305, 2001.
58. Hornick, J. L., Khawli, L. A., Hu, P., Lynch, M., Anderson, P. M., and Epstein, A. L. Chimeric CLL-1 antibody fusion proteins containing granulocyte macrophage colony-stimulating factor or interleukin-1 with specificity for B-cell malignancies exhibit enhanced effector functions while retaining tumor targeting properties. Blood 89: 4437-4447, 1997.
59. Uchida J, Hamaguchi Y, Oliver JA, Ravetch JV, Poe JC, Haas KM, Tedder TF. The Innate Mononuclear Phagocyte Network Depletes B Lymphocytes through Fc Receptor-dependent Mechanisms during Anti-CD20 Antibody Immunotherapy. J Exp Med. 2004;199:1659-1669
60. Clynes RA, Towers TL, Presta LG, Ravetch JV. Inhibitory Fc receptors modulate in vivo cytoxicity against tumor targets. Nat Med. 2000;6:443-446
61. CamiMeri-Broet S, Mounier N, Delmer A, Briere J, Casasnovas O, Cassard L, Gaulard P, Christian B, Coiffier B, Sautes-Fridman C. FcgammaRIIB expression in diffuse large B-cell lymphomas does not alter the response to CHOP+Rituximab (R-CHOP). Leukemia. 2004:18:2038-2040
62. Weng WK, Levy R. Two immunoglobulin G fragment C receptor polymorphisms independently predict response to Rituximab in patients with follicular lymphoma. J Clin Oncol. 2003;21:3940-3947.

## Claims

1. A cancer therapeutic agent which is made up of a polymer to which is linked at least 5 anti-CD20 antibodies, wherein said antibodies comprise Rituximab, and wherein said antibodies can bind to a tumor cell CD20 antigen and induce apoptosis or cell death of the tumor cell, wherein the cancer therapeutic agent is not a liposome or part of a liposome, and wherein said polymer is selected from the group consisting of dextran, block copolymers of poly(ethylene glycol)s (PEG), polyamidoamine dendrimers (PAPAM), synthetic copolymers of N-(2-hydroxypropyl)methacrylamide (HPMA), and nondegradable copolymers of N-(2-hydroxypropyl)methacrylamide (PHPMA).

2. A pharmaceutical composition comprising a cancer therapeutic agent according to claim 1.

3. The pharmaceutical composition, of claim 2 wherein said antibodies comprise two or more different antibodies which bind to different epitopes of the same tumor antigen.

4. The pharmaceutical composition of claim 2, wherein at least one antibody is a bivalent antibody.

5. The pharmaceutical composition of claim 2, wherein at least one antibody is a monovalent antibody.

6. The pharmaceutical composition of claim 2, wherein at least one antibody is a fragment of an antibody.

7. The pharmaceutical composition of any of claims 2-6 wherein the pharmaceutical composition further comprises a second antibody selected from the group consisting of anti-CD19 antibody, anti-CD20 antibody, anti-CD21 antibody, anti-HER-2, and anti-CD22 antibody.

8. The pharmaceutical composition of claim 7 wherein the antibody is anti-HER-2.

9. The pharmaceutical composition of any of claims 2 to 8 wherein the pharmaceutical composition comprises at least 10 anti-CD20 antibodies, wherein said antibodies comprise Rituximab.

10. The pharmaceutical composition of any of claims 2-9 wherein said tumor is a lymphoma or a leukemia.

11. The pharmaceutical composition of any of claims 2-10 wherein said polymer is at least 1,000 daltons in size.

12. The pharmaceutical composition of any of claims 2-11 wherein said polymer is between 6000-8000 daltons in size.

13. The pharmaceutical composition of any of claims 2 - 12 wherein said polymer is a soluble polymer.

14. The pharmaceutical composition of any of claims 2 - 13 wherein said polymer is a linear polymer.

15. The pharmaceutical composition of any of claims 2 - 13 wherein said polymer is a branched polymer.

16. The pharmaceutical composition of any of claims 2 - 15 for use in the treatment of a tumor or cancer, wherein in the treatment the size of the tumor is reduced or the growth of cancer cells in an individual is inhibited, wherein said pharmaceutical composition binds to said tumor or cancer cells in the individual.

17. The pharmaceutical composition of claim 16 for use according to claim 16, wherein said tumor or said cancer cells are a lymphoma or a leukemia.

18. The pharmaceutical composition of claim 17 for use according to claim 17 wherein said individual exhibits a reduced level of antibody dependent cell mediated cytotoxicity, as compared to that of a normal age-matched individual.

19. The pharmaceutical composition of claim 2 for use in the treatment of metastatic cancer, wherein in the treatment the development of metastatic cancer is reduced or inhibited in an individual suffering from cancer , wherein said individual has cancer cells which expresses a tumor cell antigen to which the antibodies of said pharmaceutical composition can bind.

20. The pharmaceutical composition of claim 19 for use according to claim 19 wherein said tumor or said cancer cells are a lymphoma or a leukemia.

21. The pharmaceutical composition of claims 19 or 20 for use according to claims 19 or 20 wherein said individual exhibits a reduced level of antibody dependent cell mediated cytotoxicity, as compared to that of a normal age-matched individual.

22. A method of identifying an anti-CD20 antibody that exhibits an enhanced ability to induce tumor cell apoptosis when at least five such antibodies are linked to a polymer wherein said polymer is selected from the group consisting of dextran, block copolymers of poly(ethylene glycol)s (PEG), polyamidoamine dendrimers (PAPAM), synthetic copolymers of N-(2-hydroxypropyl)methacrylamide (HPMA), and nondegradable copolymers of N-(2-hydroxypropyl)methacrylamide (PHPMA) compared to the antibody not linked to said polymer, said method comprising:
a) linking a candidate antibody for which such testing is desired to insoluble particles and contacting said antibody linked particles to tumor cells which express an antigen to which the candidate antibody can bind; and
b) following step a) determining the extent of apoptosis induced in said tumor cells and comparing it with the extent of apoptosis induced in a separate batch of the same tumor cells contacted with the candidate antibody unlinked to the particles.

23. The method of claim 22, wherein said enhanced ability to induce tumor cell apoptosis is an increase of at least two fold using the antibody linked particles compared to the antibody unlinked to the particles.

24. The method of claims 22 or 23, wherein said insoluble particles are beads with a diameter of between 2 to 5 microns in length.

## Patentansprüche

1. Ein Krebstherapie-Wirkstoff, der aus einem Polymer besteht, an das mindestens fünf Anti-CD20 Antikörper gebunden sind, wobei die Antikörper Rituximab umfassen und wobei die Antikörper an ein Tumorzell-CD20 Antigen binden können und Apoptose oder Zelltod der Tumorzelle induzieren, wobei der Krebstherapie-Wirkstoff kein Liposom oder Teil eines Liposoms ist und wobei das Polymer ausgewählt wird aus der Gruppe bestehend aus Dextran, Block-Copolymeren aus Poly(ethylenglykol)en (PEG), Polyamidoamin Dendrimeren (PAPAM), synthetischen Copolymeren aus N-(2-Hydroxypropyl)methacrylamid (HPMA) und nicht-abbaubaren Copolymeren aus N-(2-Hydroxypropyl)methacrylamid (PHPMA).

2. Eine pharmazeutische Zusammensetzung, die einen Krebstherapie-Wirkstoff nach Anspruch 1 umfasst.

3. Die pharmazeutische Zusammensetzung nach Anspruch 2, wobei die Antikörper zwei oder mehr verschiedene Antikörper umfassen, die an verschiedene Epitope desselben Tumorantigens binden.

4. Die pharmazeutische Zusammensetzung nach Anspruch 2, wobei mindestens ein Antikörper ein bivalenter Antikörper ist.

5. Die pharmazeutische Zusammensetzung nach Anspruch 2, wobei mindestens ein Antikörper ein monovalenter Antikörper ist.

6. Die pharmazeutische Zusammensetzung nach Anspruch 2, wobei mindestens ein Antikörper ein Fragment eines Antikörpers ist.

7. Die pharmazeutische Zusammensetzung nach einem der Ansprüche 2-6, wobei die pharmazeutische Zusammensetzung ferner einen zweiten Antikörper umfasst, der ausgewählt wird aus der Gruppe bestehend aus Anti-CD19 Antikörper, Anti-CD20 Antikörper, Anti-CD21 Antikörper, Anti-HER-2 und Anti-CD22 Antikörper.

8. Die pharmazeutische Zusammensetzung nach Anspruch 7, wobei der Antikörper Anti-HER-2 ist.

9. Die pharmazeutische Zusammensetzung nach einem der Ansprüche 2 bis 8, wobei die pharmazeutische Zusammensetzung mindestens 10 Anti-CD20 Antikörper umfasst, wobei die Antikörper Rituximab umfassen.

10. Die pharmazeutische Zusammensetzung nach einem der Ansprüche 2-9, wobei der Tumor ein Lymphom oder eine Leukämie ist.

11. Die pharmazeutische Zusammensetzung nach einem der Ansprüche 2-10, wobei das Polymer mindestens eine Größe von 1.000 Dalton besitzt.

12. Die pharmazeutische Zusammensetzung nach einem der Ansprüche 2-11, wobei das Polymer eine Größe zwischen 6000-8000 Dalton besitzt.

13. Die pharmazeutische Zusammensetzung nach einem der Ansprüche 2-12, wobei das Polymer ein lösliches Polymer ist.

14. Die pharmazeutische Zusammensetzung nach einem der Ansprüche 2-13, wobei das Polymer ein lineares Polymer ist.

15. Die pharmazeutische Zusammensetzung nach einem der Ansprüche 2-13, wobei das Polymer ein verzweigtes Polymer ist.

16. Die pharmazeutische Zusammensetzung nach einem der Ansprüche 2-15 zur Verwendung bei der Behandlung eines Tumors oder Krebs, wobei durch die Behandlung die Größe des Tumors reduziert oder das Wachstum der Krebszellen in einem Individuum gehemmt wird, wobei die pharmazeutische Zusammensetzung an den Tumor oder die Krebszellen in dem Individuum bindet.

17. Die pharmazeutische Zusammensetzung nach Anspruch 16 zur Verwendung gemäß Anspruch 16, wobei der Tumor oder die Krebszellen ein Lymphom oder eine Leukämie sind.

18. Die pharmazeutische Zusammensetzung nach Anspruch 17 zur Verwendung gemäß Anspruch 17, wobei das Individuum einen reduzierten Spiegel Antikörper-abhängiger, Zell-vermittelter Zytotoxizität im Vergleich zu dem eines normalen Individuums gleichen Alters aufweist.

19. Die pharmazeutische Zusammensetzung nach Anspruch 2 zur Verwendung bei der Behandlung von metastasierendem Krebs, wobei durch die Behandlung die Entwicklung von metastasierendem Krebs in einem Individuum, das unter Krebs leidet, reduziert oder inhibiert wird, wobei das Individuum Krebszellen besitzt, die ein Tumorzell-Antigen exprimieren, an das die Antikörper der pharmazeutischen Zusammensetzung binden können.

20. Die pharmazeutische Zusammensetzung nach Anspruch 19 zur Verwendung gemäß Anspruch 19, wobei der Tumor oder die Krebszellen ein Lymphom oder eine Leukämie sind.

21. Die pharmazeutische Zusammensetzung nach Anspruch 19 oder 20 zur Verwendung nach Anspruch 19 oder 20, wobei das Individuum einen reduzierten Spiegel an Antikörper-abhängiger; Zell-vermittelter Zytotoxizität im Vergleich zu dem eines normalen Individuums gleichen Alters aufweist.

22. Ein Verfahren zur Identifizierung eines Anti-CD20 Antikörpers, der eine gesteigerte Fähigkeit besitzt, Tumorzell-Apoptose zu induzieren, wenn mindestens fünf solcher Antikörper an ein Polymer gebunden sind, wobei das Polymer ausgewählt wird aus der Gruppe bestehend aus Dextran, Block-Copolymeren aus Poly(ethylenglycol)en (PEG), Polyamidoamin Dendrimeren (PAPAM), synthetischen Copolymeren aus N-(2-Hydroxypropyl)methacrylamid (HPMA) und nicht abbaubaren Copolymeren aus N-(2-Hydroxypropyl)methacrylamid (PHPMA), im Vergleich zu dem Antikörper, der nicht an das Polymer gebunden ist, wobei das Verfahren umfasst:
a) Binden eines Kandidatenantikörpers, für den ein solches Testverfahren gewünscht ist, an unlösliche Partikel und in Kontakt bringen des Antikörper-gebundenen Partikels mit Tumorzellen, die ein Antigen exprimieren, an das der Kandidatenantikörper binden kann; und
b) anschließend an Schritt a) Bestimmen des Ausmaßes an Apoptose, die in den Tumorzellen induziert wird, und Vergleichen dieses mit dem Ausmaß an Apoptose, das in einer separaten Probe der gleichen Tumorzellen induziert wird, die mit dem Kandidatenantikörper, der nicht an die Partikel gebunden ist, in Kontakt gebracht wurde.

23. Das Verfahren nach Anspruch 22, wobei die erhöhte Fähigkeit, unter Verwendung der Antikörper-gebundenen Partikeln, Tumorzell Apoptose zu induzieren, im Vergleich zu Antikörpern, die nicht an den Partikeln gebunden sind, eine mindestens zweifache Erhöhung ist.

24. Das Verfahren nach den Ansprüchen 22 oder 23, wobei die unlöslichen Partikel Kügelchen mit einem Durchmesser von zwischen 2 bis 5 Mikrometeren Länge sind.

## Revendications

1. Agent thérapeutique anticancéreux qui est constitué d'un polymère auquel est lié au moins 5 anticorps anti-CD20, lesdits anticorps comprenant du Rituximab, et lesdits anticorps pouvant se lier à un antigène CD20 de cellules tumorales et induire l'apoptose ou la mort cellulaire de la cellule tumorale, où l'agent thérapeutique anticancéreux n'est pas un liposome ou une partie d'un liposome, et où ledit polymère est choisi parmi le groupe consistant en dextrane, copolymères blocs de poly(éthylène glycol) (PEG), dendrimères de polyamidoamine (PAPAM), copolymères synthétiques de N-(2-hydroxypropyl)méthacrylamide (HPMA), et copolymères non dégradables de N-(2-hydroxypropyl)méthacrylamide (PHPMA).

2. Composition pharmaceutique comprenant un agent thérapeutique anticancéreux selon la revendication 1.

3. Composition pharmaceutique selon la revendication 2, dans laquelle lesdits anticorps comprennent deux ou plusieurs anticorps différents qui se lient à différents épitopes du même antigène tumoral.

4. Composition pharmaceutique selon la revendication 2, dans laquelle au moins un anticorps est un anticorps bivalent.

5. Composition pharmaceutique selon la revendication 2, dans laquelle au moins un anticorps est un anticorps monovalent.

6. Composition pharmaceutique selon la revendication 2, dans laquelle au moins un anticorps est un fragment d'un anticorps.

7. Composition pharmaceutique selon l'une quelconque des revendications 2 à 6, laquelle composition pharmaceutique comprend en outre un deuxième anticorps choisi parmi le groupe consistant en anticorps anti-CD 19, anticorps anti-CD20, anticorps anti-CD21, anti-HER-2, et anticorps anti-CD22.

8. Composition pharmaceutique selon la revendication 7, dans laquelle l'anticorps est anti-HER-2.

9. Composition pharmaceutique selon l'une quelconque des revendications 2 à 8, laquelle composition pharmaceutique comprend au moins 10 anticorps anti-CD20, où lesdits anticorps comprennent du Rituximab.

10. Composition pharmaceutique selon l'une quelconque des revendications 2 à 9, dans laquelle ladite tumeur est un lymphome ou une leucémie.

11. Composition pharmaceutique selon l'une quelconque des revendications 2 à 10, dans laquelle ledit polymère a au moins une taille de 1000 daltons

12. Composition pharmaceutique selon l'une quelconque des revendications 2 à 11, dans laquelle ledit polymère a une taille comprise entre 6000 et 8000 daltons.

13. Composition pharmaceutique selon l'une quelconque des revendications 2 à 12, dans laquelle ledit polymère est un polymère soluble.

14. Composition pharmaceutique selon l'une quelconque des revendications 2 à 13, dans laquelle ledit polymère est un polymère linéaire.

15. Composition pharmaceutique selon l'une quelconque des revendications 2 à 13, dans laquelle ledit polymère est un polymère ramifié.

16. Composition pharmaceutique selon l'une quelconque des revendications 2 à 15 destinée à une utilisation dans le traitement d'une tumeur ou d'un cancer, où dans le traitement la taille de la tumeur est réduite ou la croissance des cellules cancéreuses chez un individu est inhibée, où ladite composition pharmaceutique se lie à ladite tumeur ou auxdites cellules cancéreuses chez l'individu.

17. Composition pharmaceutique selon la revendication 16 destinée à une utilisation selon la revendication 16, où ladite tumeur ou lesdites cellules cancéreuses sont un lymphome ou une leucémie.

18. Composition pharmaceutique selon la revendication 17 destinée à une utilisation selon la revendication 17, où ledit individu présente un niveau réduit de cytotoxicité cellulaire dépendant des anticorps, comparé à celui d'un individu normal d'âge correspondant.

19. Composition pharmaceutique selon la revendication 2 destinée à une utilisation dans le traitement d'un cancer métastasique, où dans le traitement le développement de cancer métastasique est réduit ou inhibé chez un individu atteint d'un cancer, où ledit individu présente des cellules cancéreuses qui expriment un antigène de cellule tumorale auquel les anticorps de ladite composition pharmaceutique peuvent se lier.

20. Composition pharmaceutique selon la revendication 19 destinée à une utilisation selon la revendication 19, où ladite tumeur ou lesdites cellules cancéreuses sont un lymphome ou une leucémie.

21. Composition pharmaceutique selon les revendication 19 ou 20 destinée à une utilisation selon les revendications 19 ou 20, où ledit individu présente un niveau réduit de cytotoxicité cellulaire dépendant des anticorps, comparé à celui d'un individu normal d'âge correspondant.

22. Procédé d'identification d'un anticorps anti-CD20 qui présente une capacité augmentée à induire l'apoptose de cellules tumorales quand au moins cinq de ces anticorps sont liés à un polymère, ledit polymère étant choisi parmi le groupe consistant en dextrane, copolymères blocs de poly(éthylène glycol) (PEG), dendrimères de polyamidoamine (PAPAM), copolymères synthétiques de N-(2-hydroxypropyl)méthacrylamide (HPMA), et copolymères non dégradables de N-(2-hydroxypropyl)méthacrylamide (PHPMA), comparé à l'anticorps qui n'est pas lié audit polymère, ledit procédé consistant à :
a) lier un anticorps candidat pour lequel un tel essai est souhaité à des particules insolubles et mettre en contact lesdites particules liés à l'anticorps avec des cellules tumorales qui expriment un antigène auquel l'anticorps candidat peut se lier ; et
b) après l'étape a) déterminer le degré d'apoptose induite dans lesdites cellules tumorales et le comparer avec le degré d'apoptose induite dans un lot séparé des mêmes cellules tumorales mises en contact avec l'anticorps candidat qui n'est pas lié aux particules.

23. Procédé selon la revendication 22, dans lequel ladite capacité augmentée à induire l'apoptose de cellules tumorales est une augmentation d'au moins deux fois en utilisant les particules liées à l'anticorps comparée à l'anticorps qui n'est pas lié aux particules.

24. Procédé selon les revendications 22 ou 23, dans lequel lesdites particules insolubles sont des perles avec un diamètre compris entre 2 à 5 microns de longueur.
